# EUROPEAN PATENT SPECIFICATION

(11) **EP 4 392 102 B1**
(45) Date of publication and mention of the grant of the patent: **09.12.2026**
(21) Application number: 21773964.8
(22) Date of filing: 27.08.2021
(51) Int. Cl.: A61M 5/315

(54) **INJECTOR DEVICE STOPPER WITH ACTIVATABLE LAYER**
INJEKTORVORRICHTUNGSANSCHLAG MIT AKTIVIERBARER SCHICHT
BOUCHON DE DISPOSITIF INJECTEUR AVEC COUCHE ACTIVABLE

(43) Date of publication of application: 03.07.2024
(73) Proprietor: W. L. Gore & Associates, Inc., Newark, DE 19711 (US)
(72) Inventor: CULLY, Edward H., Newark, Minnesota 19711 (US); HARDIE, William G., Newark, Delaware 19711 (US)
(74) Representative: HGF
(86) International application number: PCT/US2021/047938
(87) International publication number: WO 2023/027722

(56) References cited:
- WO-A1-2020/118275
- US-A1- 2011 137 263
- US-A1- 2016 287 800
- US-A1- 2017 021 107

## Description

### FIELD

Various inventive concepts addressed in this description relate to injector devices, such as syringes, auto-injectors, and pens, that include a barrel and a stopper slidably received in the barrel, as well as associated methods of making and using such devices.

### BACKGROUND

Injector devices (e.g., syringes, auto-injectors and pens) typically include a barrel, a stopper positioned within the barrel, and a plunger rod or actuation mechanism to displace the stopper. The stopper is typically air and liquid impermeable and forms an air and liquid tight seal with the barrel while also possessing low-friction slidability. Air impermeability and liquid impermeability are important for eliminating liquid leakage within the barrel and the introduction of air between an outer face of the stopper and an inner wall of the barrel when charging or discharging the liquid inside the injector device. Low-friction slidability is important for facilitating the charging and discharging of the liquid inside the injector device. In addition to these requirements, a medical syringe, auto-injector, or pen should not adversely affect any pharmaceutical composition such as biopharmaceuticals that come in contact with the syringe (e.g., a pre-filled syringe, auto-injector, or pen comprising a pharmaceutical composition).

Some examples of injector device components can be found in U.S. Publication 2021/0030970 by Applicant W. L. Gore & Associates, Inc. entitled, "Medical Injector devices Having Low Lubricant Hydrophobic Syringe Barrels," which describes medical injector devices that include a barrel having an inner surface that is hydrophobic. The medical injector device includes a barrel and a stopper that can provide air and liquid impermeability while also possessing on or more of a low break loose force, a low average glide force, and a low glide force variation.

Additional examples of injector device components can be found in U.S. Patent 8,722,178, and 9,597,458 and U.S. Publication 2016/0022918, each by Applicant W. L. Gore & Associates, Inc. and entitled, "Syringe Stoppers," "Fluoropolymer Barrier Materials for Containers," and "Non-Fluoropolymer Barrier Materials for Containers," respectively (e.g., describing syringe stoppers suitable for use in syringes without silicone oil or other liquid lubricants).

Still more examples of injector device components can be found in U.S. Patent 10,751,473 by Applicant Sumitomo Rubber Industries, Ltd. entitled, "Gasket, and Medical Syringe," which describes gaskets used for a medical syringe that include a body made of an elastic material and an inert resin film provided on a surface of the body. The gasket has a cylindrical shape, and includes annular ribs provided on an outer circumferential surface thereof, each having a sliding contact portion to be kept in sliding contact with an inner peripheral surface of a syringe barrel. The annular ribs are axially arranged from a distal end to a rear end of the gasket. The sliding contact portion of a distal annular rib has a width that is 1 to 25% of axial length of the cylindrical gasket. US2011/137263 A1 describes a syringe stopper having a low friction barrier between an elastomeric stopper material and a therapeutic in the syringe. The barrier may inhibit materials from leaching from the elastomer material or from extraction of compounds from medicants by the elastomer. A process is also described that allows for molding thin barrier layers while allowing adequate bonding with the elastomer. US 2017/021107 discloses a gasket having a lamination film thereon that is laser etched to form a groove.

### SUMMARY

Forming a durable seal can be difficult for any stopper that includes a barrier, or barrier layer, and does not use silicone or other, additional lubricious material (e.g., liquid lubricant) to fill in defects in the barrier. These defects can be caused by wrinkles that form in the barrier due to compression of the stopper during insertion, from scratches in the surface of the sealing area that occur during manufacturing or insertion of the stopper, or other defects resulting from the component manufacturing and assembly processes. It is contemplated that the addition of micro features in the sealing area of the stopper can have a dramatic effect in reducing or eliminating these sealing defects by reducing wrinkles and/or helping concentrate sealing forces in a small area to help better seal off any leakage channels associated with such defects.

Often times, defects are not created, or do not become apparent, until the stopper is inserted into the barrel. Therefore, it may not be possible to prevent, eliminate, or treat various defects prior to the stopper insertion process into the barrel. Various inventive concepts addressed in this description relate to treating such defects or improving sealing geometry during or after a stopper has been through an associated insertion process into a barrel.

Aspects of the invention are set out in claims 1 and 13. Selected further features are set out in the dependent claims. A stopper for use in an injector device, has an outer side configured for engagement with an interior bore of an injector device barrel, and includes an elastomer body and a barrier coupled to the elastomer body, the barrier having an inner surface oriented toward the elastomer body and an outer surface oriented away from the elastomer body, the barrier including a first layer of a first material and a second layer of a second material, the first layer being configured to be activatable by an energy source and the second layer configured to be less activatable by the energy source than the first layer. The barrier has at least one micro feature formed in the barrier by activating the first layer with the energy source to cause a change in volume of the first layer upon being activated by the energy source, the at least one micro feature including one or both of: a micro groove and/or a micro rib. The energy source optionally includes at least one of a laser energy source, an RF energy source, a vibrational energy source such as an ultrasonic energy source, and a thermal energy source. The first layer may be positioned under the second layer. The barrier may have a thickness between 1 µm and 200 µm. In some examples, the first material and/or the second material includes a fluoropolymer (e.g., polytetrafluoroethylene (PTFE) or expanded PTFE (ePTFE)). The first layer is optionally microporous and defines a first porosity and the second layer has a lower porosity than the first layer. And, the second layer is optionally characterized by a higher melt temperature than the first layer. The second layer may be characterized by a higher dimensional stability than the first layer. As applicable, the micro groove may have a depth from 0.25 µm to 50 µm, and optionally from 0.25 µm to 0.5 µm and a width from 0.25 µm to 50 µm, and optionally from 0.25 µm to 0.5 µm and/or the micro rib may have a height from 0.25 µm to 50 µm, and optionally from 0.25 µm to 0.5 µm and a width from 0.25 µm to 50 µm, and optionally from 0.25 µm to 0.5 µm.

In any of the preceding designs, the micro groove and/or the micro rib may extend in a circumferential direction, including, a helical direction. The micro groove may have a base and two sides, where one of the two sides defines the micro rib. Material forming the micro rib optionally has a higher density than material forming the base of the micro groove or a lower density than material forming the base of the microgroove. In some cases, the first layer includes one or more of: a material configured to increase in volume upon being activated by the energy source, and further wherein the micro rib corresponds to a portion of the first layer that has been increased in volume by being activated by the energy source and/or a material configured to be removed upon being activated by the energy source, and further wherein the micro groove corresponds to a portion of the first layer that has been removed by being activated by the energy source.

If desired, the barrier may be bonded to the elastomer body by activating the first layer with the energy source. The micro groove and/or micro rib may be formed following coupling of the barrier to the elastomer body. The porosity of a portion of the first layer corresponding to the micro groove and/or micro rib has optionally been modified following coupling of the barrier to the elastomer body. In any of the aforementioned designs, at least one of the first material of the first layer and the second material of the second layer includes a thermoplastic material and/or the first material of the first layer includes a filler configured to increase absorption of light energy and/or radiofrequency energy of the first material (e.g., the filler may include at least one of fluorinated ethylene propylene (FEP) and ethylene tetrafluoroethylene (ETFE)). For the avoidance of doubt, the micro feature may be formed on the outer surface of the barrier and/or on the inner surface of the barrier.

In any of the examples and designs mentioned above, a method of forming the injector device stopper may include forming at least one of the micro rib and/or the micro groove by activating the first layer with the energy source.

In some examples, a stopper for use in an injector device, the stopper having an outer side configured for engagement with an interior bore of an injector device barrel, includes an elastomer body and a multi-zone barrier coupled to the elastomer body. The multi-zone barrier includes a first zone having a first material property and a second zone having a second material property, the second zone being configured to be activatable by an energy source and the first zone configured to be less activatable by the energy source than the first zone. And, optionally, the multi-zone barrier has at least one micro feature formed by activating the second zone with the energy source, the at least one micro feature including one or both of: a micro groove and/or a micro rib.

In accordance with claim 13 there is provided a method of making a stopper for use in an injector device. The stopper has a seal surface configured for engagement with an interior bore of an injector device barrel. The method includes activating a first layer of a barrier of the stopper with an energy source to cause a change in volume of the first layer and form at least one micro feature, the barrier having an inner surface and an outer surface. The micro feature includes one or both of: a micro groove and/or a micro rib. The barrier includes a first layer of a first material and a second layer of a second material. And, the method also includes coupling the barrier to an elastomer body. In the method, the second layer is optionally positioned over the first layer, where the first layer is activated through the second layer. The energy source optionally includes at least one of a laser energy source, an RF energy source and a thermal energy source. In some examples, the first layer includes FEP and the second layer includes PTFE. The barrier may be coupled to the elastomer body during formation of the at least one micro feature.

Forming the at least one micro feature may include cooling the barrier after activating the first layer. And, the micro groove and micro rib may be simultaneously formed, optionally by causing melted portions of the barrier to reflow and resolidify. Activating a first layer of the barrier of the stopper with the energy source to form at least one micro feature optionally includes inducing relative movement between the energy source and the stopper, where the movement optionally includes one or both of linear movement or rotational movement.

The at least one micro feature may be formed prior to coupling the barrier to the elastomer body (optionally, with the micro feature being formed with the barrier in sheet form) or after coupling the barrier to the elastomer body. In some examples, the micro feature is formed on the outer surface of the barrier and/or on the inner surface of the barrier.

Some examples of a method of making a stopper for use in an injector device, the stopper having a seal surface configured for engagement with an interior bore of an injector device barrel, include activating a first layer of a multi-zone barrier of the stopper with an energy source to form at least one micro feature in the multi-zone barrier, the multi-zone barrier including a first zone having a first material property and a second zone having a second material property, the second zone being configured to be activatable by the energy source and the first zone configured to be less activatable by the energy source than the first zone and coupling the multi-zone barrier to an elastomer body.

Some examples of a stopper for use in an injector device, the stopper having an outer side configured for engagement with an interior bore of a barrel, include an elastomer body and a multi-layer barrier coupled to the elastomer body, the multi-layer barrier including a first layer and a second layer, the second layer having one or more discontinuous portions and the first layer extending across the one or more discontinuous portions and the elastomer body. The one or more discontinuous portions of the second layer are optionally defined by at least one micro groove and the first layer optionally provides an uninterrupted barrier between elastomer body and the at least one micro groove. The first layer may be exposed through the second layer to define at least a portion of the outer side of the stopper. The one or more discontinuous portions may result in the second layer being less resistant to tearing than the first layer at the one or more discontinuous portions.

In any of the preceding designs, the first layer may be formed of a microporous layer having a greater strength than the second layer where the first layer extends across the one or more discontinuous portions. For example, the first layer may include a densified fluoropolymer, a thermoplastic material, and/or an elastomeric material. The first layer optionally includes a micro rib and/or a microgroove. And, the discontinuous portion of the second layer optionally includes a micro rib and/or a micro groove. The second layer may be non-porous and/or made of polytetrafluoroethylene, for example.

The foregoing Examples are just that, and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 shows an injector device configured as a syringe, according to some embodiments.
FIG. 2 shows an injector device configured as an auto-injector, according to some embodiments.
FIG. 3 shows a stopper of the injector device of FIGS. 1 or 2, according to some embodiments.
FIG. 4 shows a stopper of the injector device of FIGS. 1 or 2, according to some embodiments.
FIG. 5 shows a portion of the stopper of FIGS. 3 or 4, according to some embodiments.
FIGS. 6 to 13 represent various micro features in the area of FIG. 5, according to some embodiments.
FIG. 14 shows a portion of the stopper of FIGS. 3 or 4, according to some embodiments.
FIGS. 15 to 18 represent various micro features in the area of FIG. 5, according to some embodiments.
FIGS. 19 to 21 represent systems and methods by which the system can be used for forming micro features of the stopper, such as those of FIGS. 6 to 13 and 15 to 18, according to some embodiments.
FIGS. 22 to 23 represent tooling and methods by which the tooling can be used for stopper assembly and coupling, according to some embodiments.
FIGS. 24 to 33 represent micro feature arrangements and configurations, such as for those of FIGS. 6 to 13 and 15 to 18, according to some embodiments.
FIG. 34 shows a portion of the stopper of FIGS. 3 or 4, according to some embodiments.

### DETAILED DESCRIPTION

### Definitions and Terminology

This disclosure is not meant to be read in a restrictive manner. For example, the terminology used in the application should be read broadly in the context of the meaning those in the field would attribute such terminology.

The use of headings is provided for ease of review of the description only, and are not meant to segregate or otherwise designate that concepts under one heading are inapplicable or otherwise unrelated to concepts under another heading. **In** fact, the opposite is intended and the description is meant to be read and interpreted as a whole, with various features and aspects of certain embodiments being applicable across and applicable to the various other embodiments described herein.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. **In** the event it is determined that individuals having ordinary skill in the relevant arts would not readily ascertain values for such reasonably small differences, the terms "about" and "approximately" can be understood to mean plus or minus 10% of the stated value.

As used herein, the terminology "activatable by an energy source" and its analogs refer to a change of state of a material, such as a change in physical and/or chemical state. One example of activation by an energy source includes a marked (i.e., clearly evident) change from a solid form (or more solid form) to a liquid form (or more liquid form). Another example of activation by an energy source includes exhibiting a marked (i.e., clearly evident) change in cross-linking or molecular weight (e.g., via cross-linking or chain scission) through exposure to an energy source. For reference, as used herein, "energy source" refers to sources of any of a variety of types of energy, including thermal, laser, radiofrequency (RF), microwave, ultraviolet, radiant, ultrasound, and others.

As used herein, the terms "barrier," "barrier construct," or the like refer to material that blocks or hinders interaction between one component (e.g., a stopper body) and another (e.g., a barrel and/or the contents of a barrel).

As used herein, the terms "elastic" and "elastomeric" refer to a material property understood with reference to stoppers employed in injector devices (e.g., in FDA-approved applications) and relates to the tendency of a material to spontaneously revert back, or recover, toward its pre-deformation shape after being dimensionally deformed (e.g., contracted, dilated, distorted, or the like).

As used herein, the term "injector device" is meant to be inclusive of any of a variety devices that include a stopper received in a barrel and an actuation mechanism configured to displace the stopper within the barrel to eject, or deliver contents held in the barrel from within the barrel. Examples of injector devices include syringes, auto-injectors, and pens.

As used herein, the term ""macro feature" (e.g., as in "macro rib" or "macro groove") is meant to denote a stopper rib or groove feature, the contours of which are visible with the naked eye, or a stopper feature that exhibits a height that is two or more times the thickness of the barrier of the stopper.

As used herein the term "micro feature" (e.g., such as a micro rib, micro groove, or micro void) is meant to denote a stopper feature (whether a surface feature or subsurface feature), the contours of which are not visible with the naked eye (though the general existence of the feature may itself be appreciable). For example, a micro feature would include a micro rib or micro groove feature of a stopper that is located on or in a macro rib or macro groove.

As used herein, the term "multi-layer barrier" refers to a barrier construct that has a plurality of layers of material, at least portions of which are arranged in a superimposed fashion one over the other (a parallel arrangement), or in some cases, one adjacent the other (a series arrangement). A multi-layer construct may have thicknesses or layers of material with relatively sharp, distinct boundaries, or may have blended or more gradual transition boundaries therebetween.

As used herein, the term "multi-zone barrier" refers to a barrier construct that has a plurality of zones, or sections having different material properties. A multi-zone construct may have zones, or sections separated by relatively sharp, distinct boundaries, or may have blended or gradual boundaries. Some examples of multi-zone barriers include distinct layers arranged in parallel or in series, such that a multi-layer barrier also defines a multi-zone barrier. Other examples may include a single layer that is modified to define multiple zones.

As used herein, the term "oscillate" and the like (e.g., "oscillation") is meant to denote motion that alternates in direction at a frequency that may be constant or varying.

As used herein, the term "proximal" means closer to the operator end of a device (e.g., plunger end) while the term distal means further away from the operator than proximal (e.g., piercing element end).

As used herein, the term "rotate" and the like (e.g., "rotation") is meant to denote circumferentially-oriented motion.

As used herein, the term "sealing surface" is meant to denote a feature that maintains a liquid-tight seal (e.g., in storage and/or in use).

As used herein, the terms "silicone" and "silicone oil" may be used interchangeably herein.

As used herein, the term "substantially free" is meant to denote an unquantifiable or trace amount of the identified substance (e.g., silicone, silicone oil, or other lubricant), or that there is not any amount intentionally added to the system (e.g., no silicone oil intentionally added to an injector device, such as the barrel or stopper).

As used herein, the term "vibrate" (e.g., "vibration") is meant to denote motion that alternates having an acceleration that alternates in direction at a frequency that may be constant or varying.

As used herein, the term "wiper" is meant to refer to an element, sometimes referred to as a "wiper element" that is mobile (e.g., flexible or bendable) and configured to rub against a surface.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

The present disclosure is directed to injector devices (e.g., syringes, auto-injectors, and pens) that include a stopper at least partially covered with a fluoropolymer or non-fluoropolymer film or fluoropolymer or non-fluoropolymer laminate, a barrel, and a plunger rod or actuation mechanism to displace the stopper within the barrel.

Various aspects of this description relate to a barrier of the stopper that has at least one micro feature formed by activating the barrier with an energy source (e.g., a laser). For example, the barrier 242 may include multiple layers, or be a multi-layer barrier, where one layer (or layers) is configured to be more reactive to the energy source than another layer (or other layers) of the construct. And, in various embodiments that will also be subsequently described, one or more micro features may be formed prior to coupling the barrier to the body of the stopper, after coupling the barrier to the body but before inserting the stopper into the barrel, and/or after coupling the barrier to the body but before inserting the stopper into the barrel 20. Various advantages may be realized leveraging such features, including more efficient and/or higher yield manufacturing, reduced contamination and/or particulate generation, enhanced sealing, or others.

### Injector Device Concepts

In use, the injector devices may be employed for storing (e.g., short term or long term) and delivering a fluid, which is typically a therapeutic or other substance delivered to a patient for medical use. In some embodiments, such injector devices may be pre-filled with a therapeutic (e.g., as a pre-filled syringe) in advance of the planned use of the injector device to deliver the therapeutic to a patient. The injector devices may contain a therapeutic that treats diseases, such as, but not limited to, ocular disease (e.g., macular degeneration and glaucoma) or diabetes. Non-limiting examples of potential therapeutics are subsequently described. Advantageously, in various embodiments, the stoppers and barrels do not contain silicone, or silicone oil. For example, the barrels and stoppers in the injector devices described herein may be free or substantially free of silicone and silicone oil (or other liquid lubricant), according to various embodiments. In some instances, the stoppers and barrels do not contain any substantial amount, or are substantially free of any other liquid lubricant (excluding, of course, therapeutic substances in the injector device that are in liquid form, and thus lubricating themselves to at least some extent).

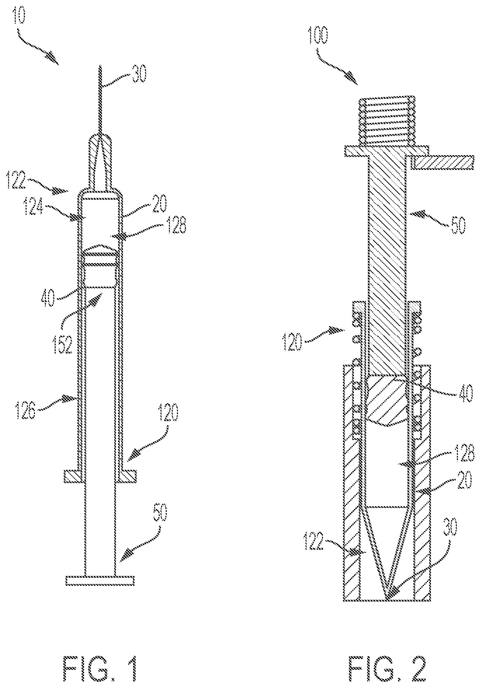

FIG. 1 depicts an injector device 10 in the form of a syringe, according to some embodiments. As shown, the injector device 10 includes a barrel 20, a piercing element 30, and a stopper 40 received in the barrel 20 and operatively coupled to an actuation mechanism 50 (e.g., a plunger rod as shown).

As shown, the barrel 20 extends between a proximal end 120 and a distal end 122. The barrel 20 has an inner surface 124 and an outer surface 126, the inner surface bounding a receiving chamber 128 defined by the barrel 20. As shown, the proximal end 120 of the barrel 20 may include a flange that may be used as a finger stopper or handle to assist a user in pressing and pulling the actuation mechanism 50.

The piercing element 30 may include a sharply pointed needle cannulae, or a blunt-ended cannula, such as those employed with "needleless" systems. For ease of illustration, the piercing element 30 is depicted as a sharply pointed, elongate needle cannula with a sharply pointed distal end. As shown, the piercing element 30 is coupled with the distal end 122 of the barrel 20.

The stopper 40 is configured to be slidably received in the barrel 20, and to seal with the inner surface 124 of the barrel 20. More specifically, the stopper 40 is configured to be actuated within the barrel 20 by the actuation mechanism 50 to pressurize and expel contents of the receiving chamber 128 from the barrel 20 through the piercing element 30.

The actuation mechanism 50 has a distal end 152 and a proximal end 154, where the distal end 152 is operatively coupled to the stopper 40, for example being fastened, integrally formed with, or otherwise associated with the stopper 40 in such a manner that the actuation mechanism 50 is configured to displace the stopper 40 within the barrel 20 in a longitudinal (or other) direction.

FIG. 2 depicts an injector device 100 in the form of an auto-injector, according to some embodiments, in which the barrel 20, the stopper 40 and the actuation mechanism 50 (also described as an injection member in association with the injector device 100) may be similarly configured and employed. The actuation mechanism 50 of the injector device 100 may be employ, or exhibit a variable actuation force that is applied to the stopper 40. For example, the actuation mechanism 50 may include one or more biasing members (e.g., springs) and other features for achieving such functionality. Various other components of the injector device 100 are substantially similarly to those of the injector device 10, as would be understood by those in the relevant field of practice. For purposes of this description, the various features of the stopper 40 described herein are applicable whether utilized in the configuration of injector device 10 or that of the injector device 100. In broader terms, the concepts described herein with respect to barrel 20 and stopper 40 may be implemented in any of a variety of injector device configurations.

The injector devices 10, 100 may include a material 60 in the receiving chamber 128 of barrel 20. In some examples, the material 60 is deposited or otherwise positioned in the chamber at a manufacturing site, or a site that is remote from the treatment site or site at which the injector device 10, 100 is to be employed by an end user (e.g., at a clinical site). In such cases, the injector device 10, 100 may be referred to as being "pre-filled" (e.g., in the example of the injector device 10, a prefilled syringe). The material 60 may be a predetermined amount (e.g., one or more doses) of a pharmaceutical composition. Some examples of suitable pharmaceutical compositions are subsequently described. However, it should be understood that the material 60 could be any type of liquid or material capable of being expelled from a syringe, or the material 60 may be all together absent from the receiving chamber, such as in an unfilled syringe. In such examples, the injector devices 10, 100 may be filled at or near a treatment site (e.g., also described as "charging" the injector device).

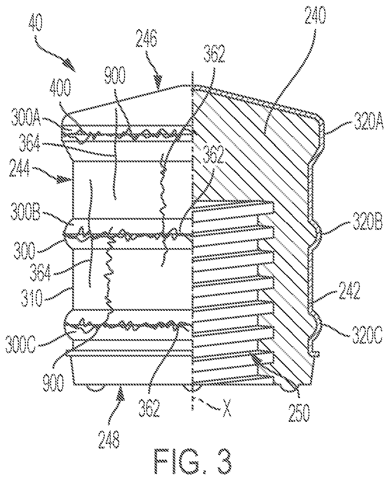

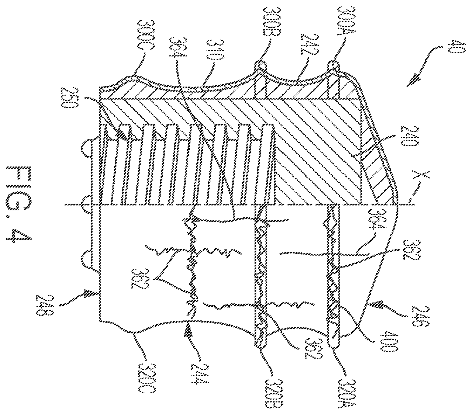

FIGS. 3 and 4 are plan, or front views of example configurations of the stopper 40, with a right half of the stopper 40 illustrated in section in the configuration of FIG. 3 and a left half of the stopper 40 illustrated in section in the configuration of FIG. 4.

As shown in each of the configurations of FIG. 3 and FIG. 4, the stopper 40 includes a body 240 made of an elastic material, and a barrier 242, such as a barrier film, provided on the body 240. The stopper 40 has an outer side 244, a longitudinal axis X, and a height along the longitudinal axis X. The stopper 40 extends between a leading face 246 and a trailing face 248. As shown, the barrier 242 may extend along a portion of (including an entirety of) the outer side 244 and/or the leading face 246. If desired, the barrier 242 may also extend along a portion of (including an entirety of) the trailing face 248.

In some embodiments, the body 240 provides a desired degree of resilient compliance to the stopper 40. For example, the body 240 may be compressed upon insertion of the stopper 40 into the barrel 20 so that the stopper 40 positively engages with the barrel 20. Suitable materials for the body 240 are described further below.

In various examples, the barrier 242 provided on the body 240 is configured to inhibit migration of substances from (or to) the body 240 through the barrier 242, reduce sliding and/or static friction between the stopper 40 and the barrel 20, and/or to enhance sealing between the stopper 40 and the barrel 20. Such features are referred to in the exemplary sense, and are not meant to be an exclusive list. The barrier 242 may be a single layer, or multiple layers. The barrier 242 may be constructed with multiple layers that have unique properties from one another and/or the barrier may include multiple layers with similar properties that are fused or otherwise coupled to form a more homogenous construct with more homogenous properties from layer-to-layer. The barrier 242 may also include composite materials (e.g., a matrix film material and a filler) serving as one or more layers of the barrier 242. Suitable materials for the barrier 242 are described further below.

As shown in each of the configurations of FIGS. 3 and 4, the stopper 40 has a short, cylindrical shape, with the leading face 246 being defined by a conical end of the stopper 40. As shown, the conical end can project away from the longitudinal axis X to define an obtuse angle. In examples where the actuation mechanism 50 is coupled to the stopper 40 using a threaded fastening arrangement, the stopper 40 may include an axial recess 250 in the trailing face 248 with female threading.

As shown, the outer side 244 of stopper 40 may define one or more ribs 300, also described as macro ribs, such as one or more circumferentially extending annular ribs 300 and/or one or more grooves 310, also described as macro grooves 310, such as one or more circumferentially extending annular grooves 310. In operation, one or more of the ribs 300 are configured to engage inner surface 124 (FIGS. 1 and 2) of the barrel 20 in sliding contact. The stopper 40 may be configured to achieve container closure integrity with high levels of gas (e.g., air) and liquid impermeability while also maintaining one or more of: acceptably low break loose force, low average glide force, and low glide force variation.

The ribs 300 can be structured in any number of configurations. For example, only the distalmost or leading rib may have a sealing surface. It is to be appreciated that the quality of a seal thus formed may be assessed by any number of methods familiar to one skilled in the art (e.g. helium leak testing). In some embodiments, multiple ribs 300 may have a sealing surface. In one or more embodiment, all of the ribs 300 having a sealing surface may have a same predefined outer diameter (e.g., measured from an apex of the respective rib with the stopper 40 in a non-compressed state). In other embodiments, each rib 300 having a sealing surface may have its own predefined outer diameter. For example, a distal or leading rib may have a predefined outer diameter and a proximal or trailing rib may have a predefined outer diameter that is between about 75% and about 99.9% of the predefined outer diameter of the distal or leading rib. Other types of rib arrangements are contemplated, such as, for example having three ribs with sealing surfaces, without departing from the scope of the present disclosure.

Although three ribs 300 are shown in FIGS. 3 and 4, it should be any number of ribs (e.g., one, two, four, ten, and so forth) are contemplated. As shown, the ribs 300 include a leading rib 300A having a sealing surface 320A (also described as a sliding contact portion 320A) configured to be in sliding contact with the inner surface 124 of the barrel 20. As shown in FIG. 3, one or more of the ribs 300 optionally has a flattened profile (e.g., the leading rib 300A) in which the sealing surface (e.g.., the sealing surface 320A) may be somewhat flattened, and have a width that is 1 to 25% of the length of the outer side 244 of the stopper 40. As shown in FIG. 4, one or more of the ribs 300 (e.g., the leading rib 300A) optionally has an outwardly convex shape, where the sealing surface (e.g., the sealing surface 320A) has a relative narrower profile. As shown in FIGS. 3 and 4, the ribs 300 also include an intermediate rib 300B and a trailing rib 300C. As shown, the intermediate rib 300B and the trailing rib 300C optionally have an outwardly convex shape as seen in section. Each of the intermediate rib 300B and trailing rib 300C optionally have sealing surfaces 320B, 320C, respectively, that are configured to be in sliding contact with the inner surface 124 of the barrel 20. Where one or more of the ribs 300 have an outwardly convex shape, the corresponding sealing surfaces may have relatively small widths as measured along the longitudinal axis X of the stopper 40. Depending upon configuration, each of the sliding contact portions 320B, 320C may have widths that are greater than 0% and up to 15% of the length of the outer side 244 of the stopper 40.

As shown in FIGS. 3 and 4, the outer side 244 of the stopper 40 may include one or more defects 900, such as wrinkles 362 and scratches 364. The various defects 900, such as the wrinkles 362 and/or scratches 364 may be oriented longitudinally, circumferentially, or both (e.g., helically). The defects 900 may be relatively linear, curved, or both. The defects may be located at any location on the stopper 40, but may be particularly prevalent on the ribs 300 and the associated sealing surfaces 320, or sliding contact surfaces 320, as well as on or along one or more micro features 400, such as those subsequently described. These defects may be formed at any point in the manufacturing process, including when the stopper 40 is first formed (e.g., when the barrier 242 is attached to the body 240) or during the process of installing the stopper 40 into the barrel 20. For example, the wrinkles 362 may be formed when the stopper is diametrically compressed. And, the scratches 364 may be formed when the stopper 40 is slid against the barrel 20 or another tubular member utilized during the assembly process, for example.

### Micro Feature Concepts

As designated in FIGS. 3 and 4, the stopper 40 includes one or more micro features 400 located at one or more of the ribs 300, such as at the sliding contact portion 320A of the leading rib 300A. In some examples, the one or more micro features 400 include one or more micro grooves and/or micro ribs. In some examples, the micro feature 400 has a width and a depth, where depth is the amount of projection in the case of a micro rib and the amount of recess in the case of a micro groove. In some embodiments, one or both of the width and the depth are not greater than 200 µm, not greater than 100 µm, not greater than 50 µm, not greater than 10 µm, or not greater than 5 µm for example, though a variety of dimensions are contemplated. Note that each of the foregoing "not greater than" ranges includes a value greater than "zero".

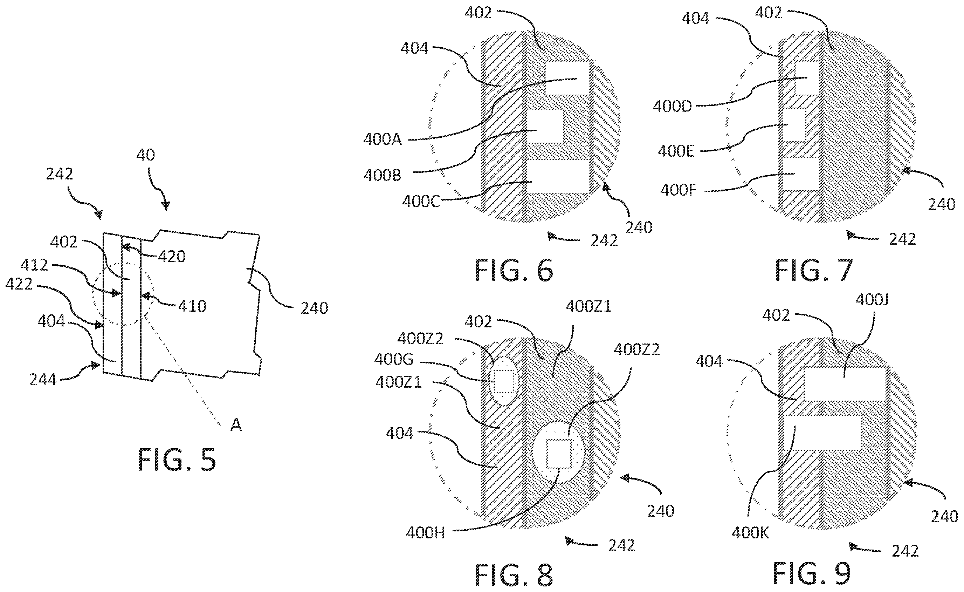

FIG. 5 is representative of an enlarged, sectional view of one or more portions of the stopper 40 along the outer side 244 of the stopper 40 (e.g., at one of the ribs 300). FIG. 6 to 9 represent various micro features (micro grooves / micro voids) included in the area "A" noted on FIG. 5 that are formed into the barrier 242. Although the body 240 and the barrier 242 are shown with straight edges in FIGS. 5-9 for ease of illustration, it should be understood that some degree of curvature may be exhibited (e.g., convex inward or outward) if the area shown corresponds to a curved portion of the stopper 40 (e.g., on one of the ribs 300).

With the foregoing in mind, FIG. 5 shows a section of the body 240 and barrier 242 of the stopper 40, according to some embodiments. As shown, the barrier 242 includes a plurality of layers, or is a multi-layer barrier including a first layer 402 of a first material and a second layer 404 of a second material. The barrier 242 may have any of a variety of thicknesses, such as between 1 µm and 200 µm.

As shown, the first layer 402 may be positioned under the second layer 404. Although two layers are generally illustrated, it should be understood that any number of layers are contemplated. As shown, the first layer 402 has an inner surface 410 facing toward the body 240 of the stopper 40 and an outer surface 412 facing toward the second layer 404. The second layer 404, in turn, includes an inner surface 420 facing toward the first layer 402 and an outer surface 422 facing away from the body 240. In various examples, the inner surface 410 of the first layer 402 is coupled (e.g., bonded, adhered, fastened, or otherwise coupled) to the body 240. And, in turn, the inner surface 420 of the second layer 404 is coupled (e.g., bonded, adhered, fastened, or otherwise coupled) to the first layer 402. In some embodiments, the first layer 402 can be referred to as an "inner layer" and the second layer 404 can be referred to as an "outer layer" of the barrier 242, although either of the first layer 402 and/or the second layer 404 may be an intermediate, or buried layer positioned between one or more other layer(s) of the barrier 242.

In various examples, one of the plurality of layers (e.g., the first layer 402) may include a first material that is more activatable by an energy source than a second material of another of the plurality of layers (e.g., the second layer 404). In particular, as illustrated in FIGS. 6 to 13, and 15 to 18, this feature of one layer being more activatable by an energy source than another may be leveraged to preferentially form a variety of micro features 400 in the barrier 242 at a variety of locations.

A variety of materials are contemplated for each layer of the barrier 242, including those separately described. For example, the first material and/or the second material may include a fluoropolymer (e.g., polytetrafluoroethylene (PTFE) or expanded PTFE (ePTFE)). In some examples, the first layer 402 is microporous and defines a first porosity and the second layer 404 has a lower porosity than the first layer, and, optionally, the second layer 404 is characterized by a higher melt temperature than the first layer 402. If desired, the second layer 404 may be characterized by a higher dimensional stability than the first layer 402. At least one of the first material of the first layer 402 and the second material of the second layer 404 may include a thermoplastic material. If desired, the first material of the first layer 402 may include a filler configured to increase absorption of light energy and/or radiofrequency energy of the first material. And, the filler may include at least one of fluorinated ethylene propylene (FEP) and ethylene tetrafluoroethylene (ETFE), for example.

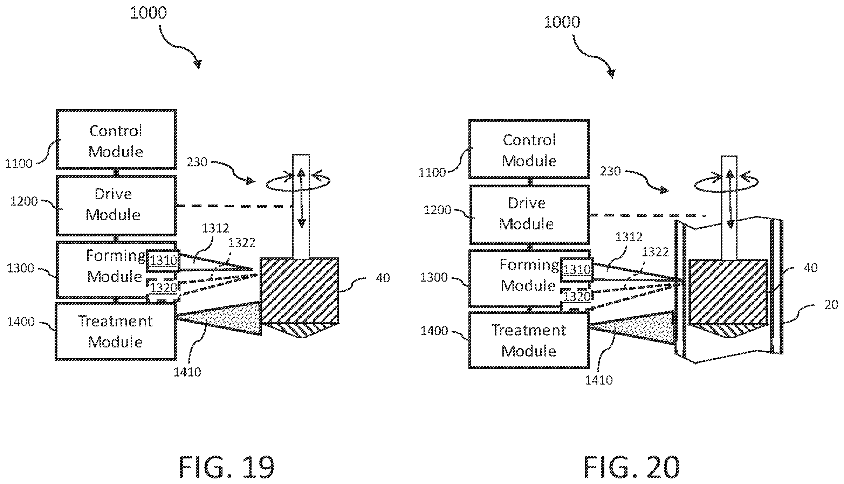

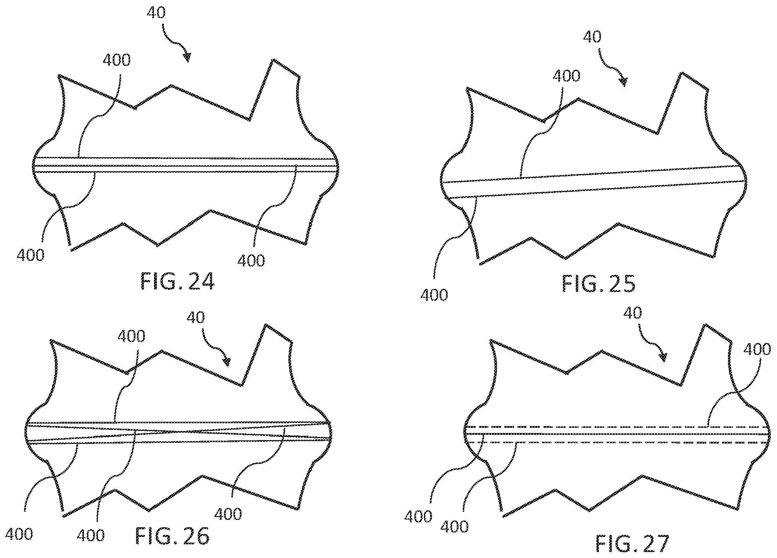

Although FIGS. 6-13 each show a set of micro feature examples (e.g., three in the case of FIG. 6), it should be understood that not all examples need be present together, and also that any of the examples may be combined with various of the other examples of micro features shown and described in association with other Figures. Example methods of forming such features would include directing an energy source (see, e.g., FIGS. 19 to 21 and associated description) through one layer (e.g., the second layer 404) into the other layer (e.g., the first layer 402) to activate a portion of the other layer (e.g., reflow, ablate, melt, or evaporate) to form the one or more micro features 400. For example, in the case of laser energy, the second layer 404 may be sufficiently transmissive to the laser to permit the laser to pass through the second layer 404 without activating the second layer 404. In turn, the first layer 402 may be relatively more absorptive to the laser energy, and thus more reactive to the laser energy. The micro features 400 be formed as a discrete volume, a continuous, annular feature extending around the stopper, and/or a series or pattern of discrete volumes (see, e.g., FIGS. 24-33 and associated description).

Following formation of the various micro features (e.g., micro voids, micro grooves, or micro ribs) at or near the particular micro feature 400 the barrier 242 generally, and the first layer 402 and/or second layer 404 more specifically, may exhibit relatively different physical properties than surrounding portions of the barrier 242, such as one or more of: increased compliance in the case of micro voids or micro grooves; reduced compression resistance in the case of micro voids or channels; increased compression resistance in the case of micro ribs, reduced thickness in the case of micro voids or channels; increased thickness in the case of micro ribs, or reduced tensile strength in the case of micro voids or micro grooves. Such characteristics may be advantageous in reducing effective sealing surface area of a rib 300 (e.g., to optimize the relationship between increased sealing force and reduced sliding resistance), creating a preferential failure line for the barrier 242 (e.g., to pre-select a more desirable area for the barrier to tear or fail to avoid contamination of the contents of injector device 10 and/or seal failure), or other advantages in performance and reliability.

In view of the foregoing, various aspects of the disclosure relate to the stopper of the injector device 10 having an outer side 244 configured for engagement with the inner surface 124 of an injector device barrel 20. The stopper 40 includes the body 240, for example formed of an elastomeric material, and the barrier 242 being coupled to the body 240. The barrier 242 has the inner surface 410 oriented toward the body 240 and an outer surface 422 oriented away from the body 240. The barrier 242 includes the first layer 402 of a first material and the second layer 404 of a second material. The first layer 402 is configured to be activatable by an energy source (e.g., laser energy source, **RF** energy source, thermal energy source, ultrasonic energy source, microwave energy source, plasma energy source, or others) and the second layer 404 is configured to be less activatable by the energy source than the first layer 402. The barrier 242 has the one or more micro features 400 formed by activating the first layer with the energy source, the one or more micro features 400 including one or both of: a micro groove extending at least partially along the outer side 244 of the stopper 40 and/or a micro rib extending at least partially along the outer side 244 of the stopper 40.

With the foregoing in mind, FIG. 6 shows a first set of examples of potential micro features 400 formed in the first layer 402 of the barrier 242 using an energy source where the first layer 402 is more activatable by the energy source than the second layer.

As shown in FIG. 6, the one or more micro features 400 may include a buried micro groove 400A, or micro void 400A extending from the inner surface 410 partially through the thickness of the first layer 402. In order to initiate activation toward the inner surface 410 of the first layer 402, the energy source could be focused (e.g., by directing two separately angled "beams" of the energy source) toward the inner surface 410 of the first layer 402. As another example, the micro groove 400A may be formed by directing the energy source at the inner surface 410 of the first layer 402 prior to coupling the barrier 242 to the body 240.

FIG. 6 shows another example of a microfeature 400 in the form of a micro groove 400B or micro void 400B extending from the outer surface 412 of the first layer 402 partially through the thickness of the first layer 402. Again, energy could be directed through the second layer 404 into the first layer 402 to activate a portion of the first layer 402 (e.g., reflow, ablate, melt, or evaporate) to form the micro groove 400B.

FIG. 6 shows still another example of a microfeature 400 in the form of a micro groove 400C or micro void 400C extending from the inner surface 410 to the outer surface 412 of the first layer 402 through the thickness of the first layer 402. As previously described, energy could be directed through the second layer 404 into the first layer 402 to activate a portion of the first layer 402 (e.g., reflow, ablate, melt, or evaporate) to form the micro groove 400B or the micro groove 400A may be formed by directing the energy source at the inner surface 410 of the first layer 402 prior to coupling the barrier 242 to the body 240.

FIG. 7 shows a second set of examples of potential micro features 400 formed in the second layer 404 of the barrier 242 using an energy source where the second layer 404 is more activatable by the energy source than the second layer.

As shown in FIG. 7, the one or more micro features 400 may include a buried micro groove 400D, or micro void 400D extending from the inner surface 420 partially through the thickness of the second layer 404. **In** order to initiate activation toward the inner surface 420 of the second layer 404, the energy source could be focused (e.g., by directing two separately angled "beams" of the energy source) toward the inner surface 420 of the second layer 404. As another example, the micro groove 400D may be formed by directing the energy source at the inner surface 410 of the first layer 402 and through the first layer 402 into the second layer 404 prior to coupling the barrier 242 to the body 240.

FIG. 7 shows another example of a microfeature 400 in the form of a micro groove 400E or micro void 400E extending from the outer surface 422 of the second layer 404 partially through the thickness of the second layer 404. Again, energy could be directed at the second layer 404 or through the first layer 402 into the second layer 404 prior to attachment of the barrier 242 to the body 240 to activate a portion of the second layer 404 (e.g., reflow, ablate, melt, or evaporate) to form the micro groove 400E.

FIG. 7 shows still another example of a microfeature 400 in the form of a micro groove 400F or micro void 400F extending from the inner surface 420 to the outer surface 422 of the second layer 404 through the thickness of the second layer 404. As previously described, energy could be directed at the second layer 404 or through the first layer 402 into the second layer 404 to activate a portion of the second layer 404 (e.g., reflow, ablate, melt, or evaporate) to form the micro groove 400F.

FIG. 8 shows a third set of examples of potential micro features 400 formed in the second layer 404 and/or the first layer 402 of the barrier 242 using an energy source where one of the first layer 402 and the second layer 404 is more activatable by the energy source than the other.

As shown in FIG. 8, the one or more micro features 400 may include a buried micro groove 400G, or micro void 400G extending between the inner surface 420 and outer surface 422 within the thickness of the second layer 404. **In** order to initiate activation within the second layer 404, the energy source could be focused (e.g., by directing two separately angled "beams" of the energy source) toward the inner surface 420 of the second layer 404 or include a localized filler material that is more absorptive to laser energy than surrounding portions of the second layer 404 (e.g., a pigment, or other material to enhance energy absorption). For example, the barrier 242 may be a multi-zone barrier including a first zone 400Z1 having a first material property (e.g., activatability or responsiveness to an energy source) and an activatable zone 400Z2 having a second material property (e.g., higher activatability or responsiveness to the energy source relative to the first zone. For example, in the case of laser energy, the first zone 400Z1 may have a lower light absorption characteristic (e.g., a lower amount or different pigment to have higher transmissivity) than the activatable zone 400Z2. As another example, the micro groove 400G may additionally or alternatively be formed by directing the energy source at the inner surface 410 of the first layer 402 and through the first layer 402 into the second layer 404 prior to coupling the barrier 242 to the body 240.

As shown in FIG. 8, the one or more micro features 400 may include a buried micro groove 400H, or micro void 400H extending between the inner surface 410 and outer surface 412 within the thickness of the first layer 402. **In** order to initiate activation within the first layer 402, the energy source could be focused (e.g., by directing two separately angled "beams" of the energy source) toward the inner surface 410 of the first layer 402 or include a localized filler material that is more absorptive to laser energy than surrounding portions of the first layer 402 (e.g., a pigment, or other material to enhance energy absorption). As another example, a buried micro groove 400H may be formed by directing the energy source at the inner surface 410 of the first layer 402 prior to coupling the barrier 242 to the body 240.

FIG. 9 shows a fourth set of examples of potential micro features 400 formed in the second layer 404 and/or the first layer 402 of the barrier 242 using an energy source where one of the first layer 402 and the second layer 404 is more activatable by the energy source than the other.

As shown in FIG. 9, the one or more micro features 400 may include a buried micro groove 400J, or micro void 400J extending between the inner surface 410 of the first layer into the second layer 404, but terminating prior to reaching the outer surface 422 of the second layer 404. Such a feature may result where the first layer 402 is more activatable by an energy source than the second layer, and as a result the micro groove 400J only forms partially through the second layer 404. The micro groove 400J may be formed using methods as previously described.

Similarly, a micro groove 400K, or micro void 400K may be formed from the outer surface 422 of the second layer 404, through the thickness of the second layer 404 and partially into the first layer 402 through the outer surface 412 of the fist layer to terminate within the thickness of the first layer 402. Such a feature may result where the first layer 402 is more activatable by an energy source than the first layer, and as a result the micro groove 400K only forms partially through the first layer 402. The micro groove 400J may be formed using methods as previously described.

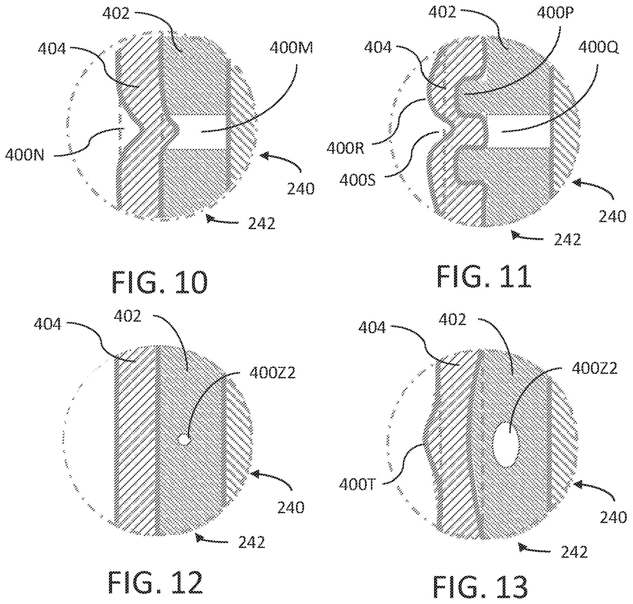

FIGS. 10 and 11 show examples of how micro features 400 may be formed in one layer (e.g., the second layer 404) through formation of microfeature 400 in another layer (e.g., the first layer 402). For example, FIG. 10 is illustrative how formation of a micro void 400M or micro groove 400M in the first layer 402 (e.g., using any of the techniques previously described) results in the formation of a micro groove 400N in the second layer 404. The first layer 402 may be more activatable or responsive to an energy source than the second layer 404, and the energy source may be used to form the micro groove 400M in the first layer 402. This, then, can be leveraged to form the micro groove 400N in the second layer 404. For example, the material of the first layer 402 may conform, or depress into the micro groove 400M to form the micro groove 400N. Use of this feature may help ensure that the micro groove 400N can be formed without defeating the integrity of the barrier 242, or in different terms, without defeating the integrity of the second layer 404 and providing a path from the outer side 244 of the stopper 40 to the body 240 of the stopper 40.

As shown in FIG. 11, micro features 400, and specifically micro ribs 400P may be formed by portions of the barrier 242 along opposite edges of other micro features 400, and specifically micro grooves 400Q or micro voids 400Q, by projections, or increased thicknesses, resulting when the micro grooves 400Q are formed by activating the barrier 242 with an energy source (e.g., laser energy). For example, as a portion of the barrier 242 (e.g., the first layer 402) is evaporated or decomposed by the energy source (e.g., laser beam) using any of the methods previously described, the evaporated or decomposed portion may be partly redeposited along the opposite edges of the micro grooves 400Q to form the micro ribs 400P.This, in turn, may result in formation of micro ribs 400R and micro grooves 400S in another layer (e.g., the second layer 404) without having to directly alter the other layer (e.g., the second layer 404) with the energy source. Such a feature may have a variety of benefits, including the avoidance of generating free particulate, contaminants or byproducts of the energy activation process that could contaminate the outer side 244 of the stopper 40, and ultimately the contents of the injector device 10.

FIGS. 12 and 13 show another example of a potential micro feature 400 in the form of a micro rib 400T (FIG. 13). As shown in FIG. 12, the first layer 402 includes a mass of material, or activatable zone 400Z2, that is configured to expand, or increase in volume via activation by an energy source (e.g., laser energy). As shown in FIG. 12, the activatable zone 400Z2 starts at a first size, or volume and following activation as shown in FIG. 13 occupies a second, larger size or volume. This expansion, or change in volume, in turn results in deflection of the barrier 242, and in particular the second layer 404, and optionally the first layer 402, resulting formation of a micro rib 400T as shown in FIG. 12. Although the expandable material may be limited to a zone, the activatable zone 400Z2, it is also contemplated that the entire layer may be formed of the activatable material and that only a portion of the layer is activated to form the micro rib 400T.

An example of an expandable, energy activatable material, can be found in U.S. Pat. 5571592 to McGregor et al. The activatable zone 400Z2 may include expandable thermoplastic microspheres interspersed and contained within the activatable zone 400Z2. The use of such expandable microspheres can allow for (1) the introduction of unexpanded microspheres into the first layer 402; and (2) expansion of the microspheres within the first layer 402 to a greater diameter. In various examples, when subjected to heat (e.g., thermal energy through application of a laser or other energy source) or similar activation energy, the microspheres dramatically expand to many times their original size and retain such size when the activation energy is removed. Processes for producing such material can be found in U.S. Pat. 3615972 to Morehouse et al., for example.

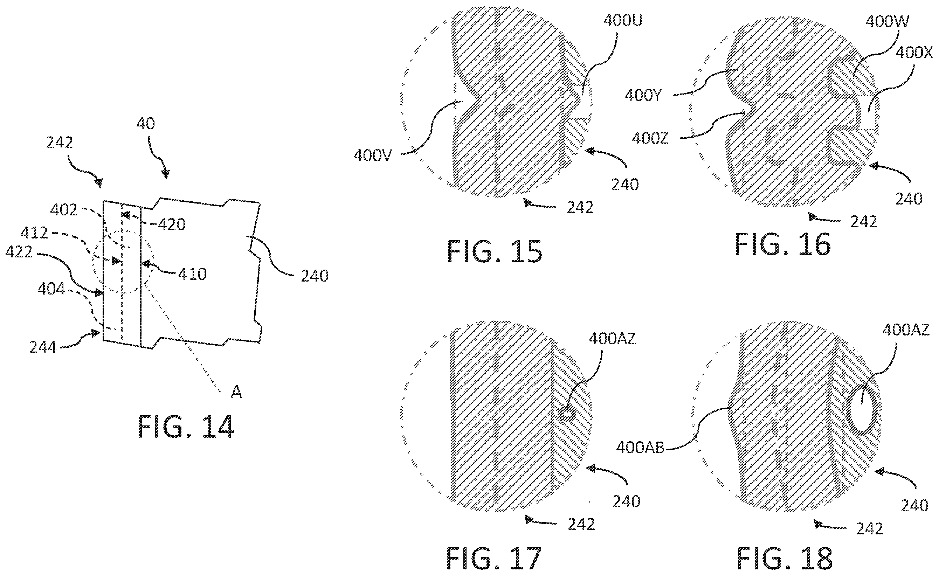

FIG. 14 is representative of an enlarged, sectional view of one or more portions of the stopper 40 along the outer side 244 of the stopper 40 (e.g., at one of the ribs 300). FIG. 15 to 18 represent various micro features (micro ribs / micro grooves / micro voids) included in the area "A" noted on FIG. 14. As shown in FIG. 14, the barrier 242 optionally includes multiple layers (as designated by the broken line in FIG. 14), but may also be a monolithic or single layer construction. In various examples, the barrier 242 is less activatable by an energy source than the body 240 of the stopper 40, and the one or more micro features 400 are formed by activating the body 240 of the stopper 40. For the avoidance of doubt, the body 240 can be considered a "layer" of the stopper 40.

FIGS. 15 and 16 show examples of how micro features 400 may be formed in one layer of the stopper 40 (e.g., the body 240) through formation of microfeature 400 in another layer (e.g., the barrier 242). For example, FIG. 15 is illustrative how formation of a micro void 400U or micro groove 400U in the body 240 (e.g., using any of the techniques previously described, including lasering "through" the barrier 242 without activating the barrier 242) results in the formation of a micro groove 400V in the barrier 242. The body 240 may be more activatable or responsive to an energy source than the barrier 242, and the energy source may be used to form the micro groove 400U in the body 240. This, then, can be leveraged to form the micro groove 400V in the barrier 242. Use of this feature may help ensure that the micro groove 400V can be formed without defeating the integrity of the barrier 242 and providing a path from the outer side 244 of the stopper 40 to the body 240 of the stopper 40.

As shown in FIG. 16, micro features 400, and specifically micro ribs 400W may be formed by portions of the barrier 242 along opposite edges of other micro features 400, and specifically micro grooves 400X or micro voids 400X, by projections, or increased thicknesses, resulting when the micro grooves 400X are formed by activating the body 240 with an energy source (e.g., laser energy). For example, as a portion of a layer of the stopper 40 (e.g., the body 240) is evaporated or decomposed by the energy source (e.g., laser beam) using any of the methods previously described, the evaporated or decomposed portion may be partly redeposited along the opposite edges of the micro grooves 400X to form the micro ribs 400W.This, in turn, may result in formation of micro ribs 400Y and micro grooves 400Z in another layer (e.g., the barrier 242) without having to directly alter the other layer (e.g., the barrier 242) with the energy source. Such a feature may have a variety of benefits, including the avoidance of generating free particulate, contaminants or byproducts of the energy activation process that could contaminate the outer side 244 of the stopper 40, and ultimately the contents of the injector device 10.

FIGS. 17 and 18 show another example of a potential micro feature 400 in the form of a micro rib 400AB (FIG. 18). As shown in FIG. 17, a first layer of the stopper, the body 240, includes a mass of material, or activatable zone 400AZ, that is configured to expand, or increase in volume via activation by an energy source (e.g., laser energy). As shown in FIG. 17, the activatable zone 400AZ starts at a first size, or volume and following activation as shown in FIG. 18 occupies a second, larger size or volume. This expansion, or change in volume, in turn results in deflection of the barrier 242, resulting formation of a micro rib 400AB as shown in FIG. 18. Although the expandable material may be limited to a zone, the activatable zone 400AZ, it is also contemplated that the entire layer may be formed of the activatable material and that only a portion of the layer is activated to form the micro rib 400AB.

In view of the foregoing, various examples include the stopper 40, and more specifically the barrier 242 defining a micro groove (e.g., any of the micro grooves shown in FIGS. 6 to 16), the barrier 242 at the micro groove being continuous and uninterrupted, and being relatively thinner than the barrier 242 is at surrounding portions of the barrier 240.

The micro groove may define a discontinuous, broken, circumferential line pattern as described in association with FIG. 27, for example. In some examples, the barrier 242 is a multi-layer barrier (e.g., two layers or more) in which the first layer 402 has one or more discontinuous portions (e.g., a continuous circumferential micro groove or a micro groove having a discontinuous, circumferential broken line pattern as described in association with FIG. 27). The second layer 404 overlies the one or more discontinuous portions, such as a micro groove. In this manner, the second layer 404 may provide an uninterrupted barrier between the body 240 and the barrel 20, and its contents. In different terms, the second layer 404 may extend across the one or more discontinuous portions of the first layer 402.

The underlying, first layer 402 may be formed of a relatively higher strength material whereas the overlying, second layer 404 may be formed of a relatively more compliant, weaker material. In this manner, the barrier 242 may be provided with a high degree of compliance on the outer surface while also exhibiting a relatively high degree of tear resistance due to the underlying, first layer 402. This feature can then also be coupled with the ability to provide a micro groove and/or micro rib that is exhibited by the second layer 404 at the outer side 244 without directly forming (e.g., mechanically or energetically) the second layer 404, creating unwanted debris and particulate (which may contaminate the barrel 20 and its contents and/or without unduly weakening the more compliant second layer 404 such that it would fail in use.

Although various examples include forming the discontinuous portion in the underlying, first layer 402, in some examples, the second layer 404 may have one or more discontinuous portions and the first layer 402 may extend across the one or more discontinuous portions, as well as the elastomer body 21, providing a barrier between the outer side 244 and the body 240 (e.g., micro groove 400F in FIG. 7). Again, the discontinuity may be defined by at least one micro groove. The first layer 402 may be exposed through the second layer 404 to define at least a portion of the outer side 244 of the stopper 40. The one or more discontinuous portions may result in the second layer 404 being less resistant to tearing than the first layer 402 at the one or more discontinuous portions.

This feature of forming a micro channel in the second layer 404 while preserving the first layer 402, or inner layer 402 may be advantageous in at least the concept that, again the underlying first layer may be relatively stronger than the outer layer and prevent tearing through both layers to expose the underlying body 242 to the barrel 20 and its contents. For example, the first layer 402 may be formed of a microporous layer having a greater strength than the second layer 404 where the first layer 402 extends across the one or more discontinuous portions. The first layer may include a densified fluoropolymer (e.g., having a relatively high tensile strength), a thermoplastic material, and/or an elastomeric material. The first layer 402 may additionally or alternatively include a micro rib and/or a microgroove.

The discontinuous portion of the second layer 404 may include a micro rib and/or a micro groove. In some examples, the second layer may be non-porous. For example, the second layer 404 may be polytetrafluoroethylene (e.g., skived PTFE).

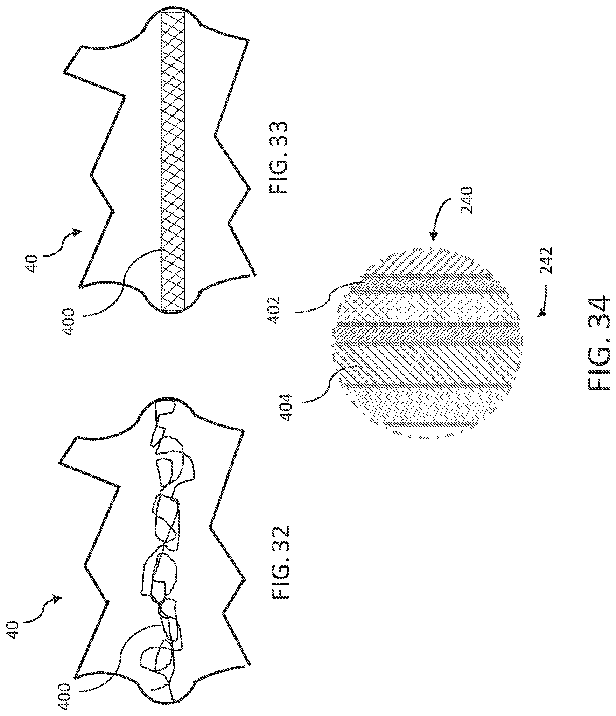

FIG. 34 is still another view of a portion of the stopper 40 corresponding to the areas "A" shown in FIGS. 5 and 12, albeit with a different barrier 242 configuration than shown in those figures. Although previously referenced, for the avoidance of doubt FIG. 35 shows an example of a multi-layer barrier configuration including more than two layers (five in total, as shown). As shown, the first layer 402 and/or the second layer 404 may be at any position within the layers. And, there may be greater or fewer layers in various implementations. The first layer 402 may be an innermost layer, or a buried layer, for example. The second layer 404 may be an outermost layer, or a buried layer, for example. And, the first layer and second layers 402, 404 may be in contact, or separated by one or more other layers.

The various micro features 400 described above may have any of a variety of dimensions. In some examples, one or more of the micro grooves have a depth from 0.25 µm to 50 µm, and optionally from 0.25 µm to 0.5 µm and a width from 0.25 µm to 50 µm, and optionally from 0.25 µm to 0.5 µm and/or one or more of the micro ribs has a height from 0.25 µm to 50 µm, and optionally from 0.25 µm to 0.5 µm and a width from 0.25 µm to 50 µm, and optionally from 0.25 µm to 0.5 µm. As will be subsequently described, the micro grooves and/or micro ribs may have any of a variety of configurations, for example extending in a circumferential direction, a helical direction, or even a longitudinal direction. As illustrated above in association with FIGS. 11 and 16, for example, one or more micro grooves may have a base and two sides, where one or both of the two sides defines a micro rib. In some embodiments, material forming the micro rib has a higher density than material forming the base of the micro groove. And, in some embodiments, material forming the micro rib has a lower density than material forming the base of the micro groove.

Also, as described above, a portion of the stopper 40, such as the first layer 402 optionally includes a material configured to increase in volume upon being activated by the energy source, and a resulting micro rib corresponds to a portion of the first layer 402 that has been increased in volume by being activated by the energy source. And, in the case of micro channels or voids, a portion of the stopper 40, such as the first layer 402 includes a material configured to be removed upon being activated by the energy source, where the micro groove corresponds to a portion of the first layer 402 that has been removed by being activated by the energy source.

### Means for Application of Energy to Stopper Components

Methods of making the stopper 40 include activating a layer or zone (e.g., the first layer 402) of the barrier 242 with an energy source to form the one or more micro features 400, the one or more micro features 400 including one or both of: a micro groove and a micro rib. The barrier 242 may be coupled to the elastomer body 240 before, or after such formation depending on the particular method. In some examples, the barrier 242 may be coupled to the body 240 during formation of the one or more micro features (e.g., by reflowing material which assists with bonding between components).

As described above, one layer (e.g., the first layer 402) can be activated by directing energy through another layer (e.g., the second layer 404). For example, the second layer 404 may be positioned over the first layer 402 and the first layer 402 can be activated through the second layer 404. In some methods, forming the at least one micro feature includes cooling the barrier 242 after activating the first layer 402. Although micro grooves and micro ribs may be separately formed, some methods include simultaneously forming one or more micro grooves and micro ribs, optionally by causing melted portions of the barrier 242 to reflow and resolidify.

Activating a layer of the barrier 242 with energy can include inducing relative movement between the energy source from the forming module 1300 and the stopper 40, the movement optionally including one or both of linear movement and/or rotational movement. The at least one micro feature 400 can be formed with the barrier in sheet form (e.g., a sheet preform) or a tubular form (e.g., a tubular preform). The micro features 400 can be formed on the outer surface 422 of the barrier 242 and/or the inner surface 410 of the barrier 242.

FIGS. 19 and 20 are illustrative of a system 1000 and a method by which the system 1000 can be used for forming one or more micro features 400 of the stopper 40. As shown, the system 1000 includes a control module 1100, a drive module 1200, a forming module 1300, and a treatment module 1400. As previously referenced, the one or more micro features 400 can be formed after assembly of the stopper 40, or prior at assembling the barrier 242 to the body 240 (e.g., by forming the micro features 400 on a barrier preform or body preform). And, as illustrated in FIG. 20, in various embodiments the one or more microfeatures may be formed in the one or more stopper components after assembly of the injector device 10 (i.e., after the stopper 40 has been inserted into the barrel 20, and optionally with the contents of the barrel 20 already in place in a pre-filled assembly).

The control module 1100 is configured to control operation of the system 1000. In various examples, the control module 1100 may include a power source (not shown), one or more microprocessors, one or more user input devices (e.g., keyboard), one or more display devices (e.g., monitor), and other features for controlling operation of the system 1000.

The power source may provide electrical power to the operative components of the control module 1100 and/or the other components of the system 1000, and may be any type of power source suitable for providing the desired performance and/or longevity requirements of the control module 1100 and/or system 1000. In various embodiments, the power source may include one or more batteries, which may be rechargeable (e.g., using an external energy source).

The control module 1100 may include, or be included in one or more Field Programmable Gate Arrays (FPGAs), one or more Programmable Logic Devices (PLDs), one or more Complex PLDs (CPLDs), one or more custom Application Specific Integrated Circuits (ASICs), one or more dedicated processors (e.g., microprocessors), one or more central processing units (CPUs), software, hardware, firmware, or any combination of these and/or other components. The control module 1100 may include a processing unit configured to communicate with memory to execute computer-executable instructions stored in the memory. Additionally, or alternatively, the control module 1100 may be configured to store information (e.g., sensed data) in the memory and/or access information (e.g., sensed data) from the memory.

In some embodiments, the memory includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory stores computer-executable instructions for causing the processor to implement aspects of embodiments of system components discussed herein and/or to perform aspects of embodiments of methods and procedures discussed herein.

The computer-executable instructions may include, for example, computer code, digital signal processing, machine-useable instructions, and the like such as, for example, program components capable of being executed by one or more processors associated with the computing device. Program components may be programmed using any number of different programming environments, including various languages, development kits, frameworks, and/or the like. Some or all of the functionality contemplated herein may also, or alternatively, be implemented in hardware and/or firmware.

In some embodiments, the drive module 1200 is controlled by the control module 1100 and produces relative motion between the forming module 1300 and one or more of the stopper components (e.g., body 240 and/or barrier 242) while the forming tool is forming the micro features 400 in a desired configuration. For example, the drive module 1200 can cause rotation of one or more of the stopper components (e.g., body 240 and/or barrier 242) with respect to the forming module 1300 and/or circumferential motion of the forming module 1300 around the stopper components. The drive module 1200 may additionally or alternatively produce axial movement of the stopper components (e.g., the body 240 and/or barrier 242). The drive module 1200 may include drive motors, sensors, control circuits, drive shafts, turn tables, and/or a variety of additional or alternative components for achieving the desired, relative motion between the forming module (and, optionally, the treatment module 1400) and the stopper components. As shown in FIG. 20, the drive module 1200 may be configured to generate relative movement between the assembled injector device 10 (e.g., the barrel 20 and stopper 40) and the forming module 1300.

The forming module 1300, which is controlled by control module 1100 in various embodiments, includes a primary energy generator 1310 that generates and directs energy 1312 to the one or more stopper components, such as the barrier 242 and/or the body 240, as previously referenced in association with FIGS. 5 to 18, for example. In some embodiments, the forming module 1300 includes a secondary energy generator 1320 that generates and directs energy 1322 to the one or more stopper components, such as the barrier 242 and/or the body 240. For example, in embodiments when present, the secondary energy generator 1320 may direct the energy 1322 at the stopper component at an angle that is offset from the energy 1312 from the primary energy generator 1310. The beams, or directionality of the two energies 1312 and 1322 may intersect at a desired location on or within the stopper component so that the cumulative energy from the energies 1312, 1322 is sufficient to activate the material of the stopper component, whereas taken alone, each of the energies 1312, 1322 would otherwise be insufficient to activate the material of the stopper component. In this manner, energy can be focused at a desired location of the stopper component (e.g., at a desired depth) as previously referenced in association with one or more of FIGS. 5 to 18, for example. The forming module preferably includes a laser energy source, although it is contemplated that any of a variety of energy sources may be implemented, including an electron beam energy source, an ultraviolet light energy source, a plasma energy source, an ultrasonic energy source, or other source of energy capable of activating the one or more stopper components.

Examples of suitable laser generators include CO2 lasers, for example. Some examples of suitable laser generators include those configured to activate material in the barrier 242 and/or body 240 without adversely impacting the barrel 20. In such examples, the choice of the type and wavelength of the laser generator may depend upon the barrel material and the stopper material. Suitable wavelengths may range between 400 to 1700 nm for barrels made of borosilicate glass, for example. In one specific example, a 1070 nm laser beam was shown to easily pass through a borosilicate barrel without heating while still delivering sufficient energy to alter stopper geometry.

In some embodiments, the forming module 1300 simultaneously forms the micro feature 400 around a circumference of the stopper (e.g., barrier 242 and/or body 240). In some embodiments, the drive module 1200 generates relative movement between the forming module 1300 and the one or more stopper components such that the beams, or directionality of the energies 1312 and/ the energy 1322 are applied to the material of the components in a desired pattern (such as a continuous circumferential pattern or any of the patterns described in association with FIGS. 24 to 33, for example. As shown in FIG. 20, in some embodiments the forming module 1300 is configured to direct energy through the barrel 20 to the stopper 40 for formation of the micro features 400. For example, the barrel 20 may be formed of optically transmissive material (e.g., borosilicate glass) and the forming module 1300 may include a laser (e.g., a CO2 laser) configured to transmit energy in the form of a laser beam through the barrel 20 to the stopper 40.

In some embodiments, treatment module 1400, which may be controlled by control module 1100, applies a treatment material 1410 to the stopper 40, such as applying a rinsing solution for removing debris generated during micro feature formation, a coolant (e.g., gas, such as nitrogen gas, or fluids, such as refrigerant) to help avoid overheating and/or encourage re-solidification of stopper component material following heating, or for other purposes. As shown in FIG. 20, the treatment module 1400 may apply treatment material 1410 to the barrel 20 (e.g., to cool the barrel 20, the stopper 40, and or contents of the barrel 20 (e.g., a therapeutic substance) during or after formation of the one or more micro features 400.

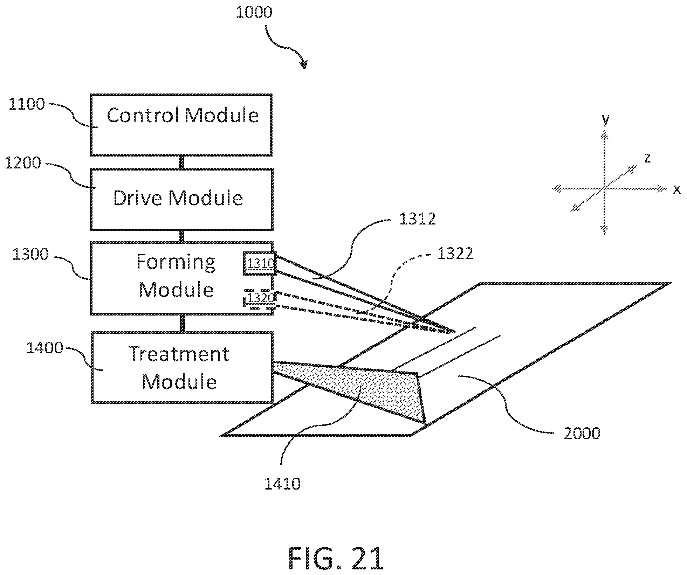

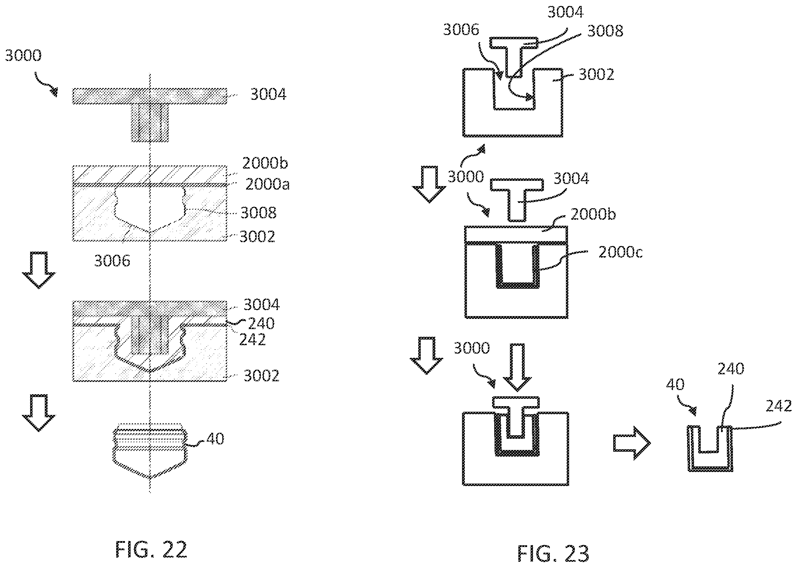

FIG. 21 shows another example of the system 1000 and a method by which the system 1000 can be used for forming one or more micro features 400 of the stopper 40, but into a preform 2000 of one or more stopper components (e.g., the body 240 or the barrier 242). For example, as will be described in association with FIGS. 22 and 23, one or more components of the stopper 40 may be provided as a preform 2000 in sheet form and then molded or otherwise assembled to form the stopper 40. The system 1000 may have largely the same components, and operate largely in a similar manner to the example of FIG. 19, with the exception that the drive module 1200 is configured to handle the preform 2000.

### Stopper Assembly and Coupling Mechanisms

Various manners of assembly the stopper, and in particular arranging the barrier 242 and the body 240 together, are contemplated.

For example, FIG. 22 includes the use of tooling 3000 similar to that to be described in connection with FIG. 23, including a mold 3002 and a forming apparatus such as mandrel 3004. The mold 3002 includes a cavity 3006 defined by an interior wall 3008. The cavity 3006 is shaped and sized to produce the stopper 40 with a desired shape and size. As shown, tooling 3000 is configured to manufacture the stopper 40 from a preform 2000a of barrier material and a preform 2000b of body material, each of the preforms 2000a, 2000b being in sheet, or relatively planar form to start.

The preforms 2000a, 2000b are optionally aligned and then forced (e.g., simultaneously) into the cavity 3006 of the mold 3002 as shown. The body 240 is thereby formed from the preform 2000b with the barrier 242 co-molded or laminated thereon from the preform 2000a to form the stopper 40 as shown. In the illustrated embodiments, the mandrel 304 is actuated to force the preforms 2000a, 2000b into the mold 3002. In some embodiments, the mandrel 3004 can be configured to define a structure in body 240 during formation (e.g., the axial recess 250 in the trailing face 248 with female threading).

Injection molding, compression molding, vacuum press molding, co-molding or other known or otherwise conventional processes and equipment can also be used to manufacture the stopper 40 using the preforms 2000a, 2000b.

As another example, FIG. 23 is illustrative of some embodiments how a preform 2000c of the material of the barrier 242 in a cylindrical form can be combined with a preform 2000b of the material of the body 240 in a sheet form to assemble the stopper 40. As shown in FIG. 23, the process includes use of tooling 3000 including a mold 3002 and a forming apparatus such as mandrel 3004. The mold 3002 includes a cavity 3006 defined by an interior wall 3008. The cavity 3006 is shaped and sized to produce the stopper 40.

Tooling 3000 is configured to manufacture the stopper 40 from the preform 2000c of barrier material and a mass body material defining the preform 2000b. As shown, the preform 2000c of barrier material is positioned in the cavity 3006 of the mold 3002. The preform 2000b of body material is then applied to the interior void area within the preform 2000c of barrier material. As shown, the mandrel 3004 is actuated to force the preform 2000b, which can be in a solid or semi-solid form, into the preform 2000c through the open proximal end portion of the preform 2000c. The mandrel 3004 can be configured to define a structure in the preform 2000b (e.g., the axial recess 250 in the trailing face 248 with female threading).

Though mandrel 3004 is optionally utilized, in other embodiments the body material is deposited into the preform 2000c of barrier material by other approaches such as in a flowable or other fluid form by the application of pressure. Injection molding, compression molding, vacuum press molding, co-molding or other known or otherwise conventional processes and equipment can be used to manufacture the stopper 40 using the preform 2000c.

Various modifications to the foregoing may be applied to enhance or achieving component bonding. In some examples, the barrier 242 may be bonded (or further bonded) to the body 240 during formation of the one or more micro features 400 or by activating the first layer 402 with the energy source. The additional use of adhesives, elastomeric bonding materials, surface treatments and other practices are also contemplated.

### Micro Feature Arrangements and Configurations

The one or more micro features 400 may be arranged in any of a variety of continuous (e.g., circumferential line) and discontinuous (e.g., broken, circumferential line) patterns. In other words, each of the one or more micro features 400 can take any of a wide variety of configurations. The various configurations and features that follow may achieve a variety of benefits and advantages. For example, the micro features 400 may be arranged to enhance sealing and/or sliding functionality of the stopper 40, reduce wrinkling of the barrier 242 (e.g., as part of compression and insertion into the barrel 20), and/or reduce the incidence of delamination or decoupling of the barrier 242 from the body 240, among others.

FIG. 24, for example, illustrates embodiments of micro features 400 that are continuous and extend about a generally linear path circumferentially around the entire outer side 244 of the stopper 40 In the embodiments shown in FIG. 24, the micro features 400 are parallel to one another and are non-intersecting, and a plane defined by each micro groove is generally orthogonal to a longitudinal axis X of the stopper 40. FIG. 25 illustrates embodiments of a stopper 40 having one or more micro features 400 (two are shown for purposes of example) located in a plane oblique to the longitudinal axis X (FIGS. 1 and 2) of the stopper 40, but otherwise similar in configuration to the micro features 400 described in connection with FIG. 24. FIG. 26 illustrates embodiments of a stopper 40 having micro features 400 defining a plurality of different oblique planes with respect to the longitudinal axis X of the stopper 40 (four such micro features 400 are shown for purposes of example). In the embodiments shown in FIG. 26, the planes and micro features 400 intersect one another. In other embodiments (not shown), one or more of the micro features 400 are in oblique and optionally parallel planes with respect to the longitudinal axis of the stopper 40 that do not intersect the planes defined by one or more other micro features 400.

Addition of the described micro features on sealing surfaces of the stopper 40 may have the advantage of enhancing sealing without increasing sliding force required to operate the injector devices. This enhanced functionality may be achieved by reduction of wrinkles formed during the assembly process (e.g., insertion of the stopper 40 into the barrel 20) and/or by altering the sealing interface by increasing the sealing pressure in micro ribs that are raised and/or reducing sliding surface areas by the addition of micro grooves.

FIGS. 27 to 29 illustrate embodiments of the stopper 40 including one or more micro features 400 that are discontinuous or broken. For example, the micro features 400 can include one or more sections comprising a depth that is about zero. Although two discontinuous micro features 400 are shown for purposes of example in FIGs. 27 to 29, other embodiments have more or fewer micro features 400 that are discontinuous. The embodiments shown in FIGS. 27 to 29, including the micro features 400, can otherwise be similar to those of described in connection with FIGS. 24 to 26, respectively.

The various broken line, or discontinuous configurations and features described above in association with FIGS. 27 to 29 may achieve a variety of benefits and advantages. The addition of discontinuous grooves or ribs can be beneficial in reducing wrinkling (e.g., micro wrinkles) that can tend to form during the insertion process when the stopper 40 is introduced into the barrel 20. For example, by arranging the micro features 400 in a discontinuous line, or pattern, the stopper 40, and in particular the barrier 242 may be less apt to wrinkle or deform when the stopper 40 is compressed for insertion into the barrel 20. For example, the pattern of micro features 400 may create strain reliefs or similar features that permit compression without (or with reduced) associated wrinkling or other unwanted deformation.

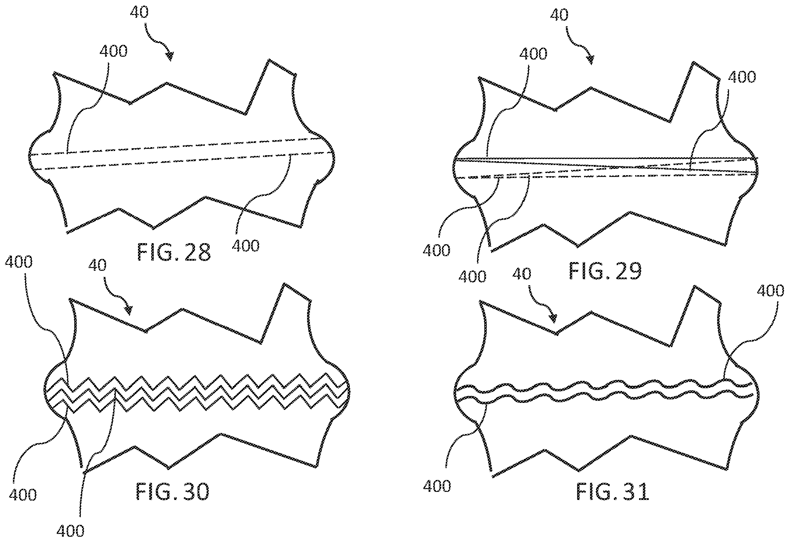

FIGS. 30 and 31 illustrates embodiments of the stopper 40 including a plurality of micro features 400 including nonlinear portions. Other embodiments include more or fewer micro features 400 including nonlinear portions such as those shown in FIGS. 30 and 31. Although the nonlinear portions of the micro features 400 of the embodiments shown in FIGS. 30 and 31 are in the form of generally repeating patterns, the nonlinear portions include or consist of non-repeating pattern portions in other embodiments. In the embodiments shown in FIGS. 30 and 31 the micro features 400 include nonlinear portions that extend completely around the stopper 40 (i.e., the micro features 400 consist of nonlinear portions). In other embodiments, one or more micro features 400 include linear and nonlinear portions.

The various non-linear configurations described above in association with FIGS. 30 and 31 may achieve a variety of benefits and advantages. For example, by arranging the micro features 400 in a non-linear configuration, the stopper 40, and in particular the barrier 242 may be less apt to wrinkle or deform when the stopper 40 is compressed for insertion into the barrel 20. For example, the undulating, or circumferentially overlapping pattern of micro features 400 may create a strain relief, gaps in the material of the barrier 242, or another effect that permits compression of the stopper 40 without (or with reduced) associated wrinkling or other unwanted deformation.

FIG. 32 illustrates embodiments of the stopper 40 including micro features 400 that extend about circuitous, nonlinear paths circumferentially around the one or more ribs 300. FIG. 33 illustrates embodiments of the stopper 40 including micro features 400 in the form of a grid or cell structure pattern. Although diamond-shaped cells are shown in FIG. 33, other embodiments include cells having other shapes. The various diamond shaped, and crossing patterns described above may also achieve a variety of benefits and advantages. Again, with such configurations, the barrier 242 may be less apt to wrinkle or deform when the stopper 40 is compressed for insertion into the barrel 20.

A variety of configurations are contemplated and embodiments of the stopper 40 may include one or more micro features 400 that each include one or more of the features or attributes of the micro grooves described above in connection with any one or more of FIGS. 24 to 33, for example.

### Exemplary Material Sets

The barrel 20 may be formed of a substantially rigid or hard material, such as a glass material (e.g., borosilicate glass), a ceramic material, one or more polymeric materials (e.g., polypropylene, polyethylene, and copolymers thereof), a metallic material, or a plastic material (e.g., cyclic olefin polymers (COC) and cyclic olefin copolymers (COP), and combinations thereof. It is to be appreciated that barrels formed of materials that are not inherently hydrophobic (e.g. a glass barrel) may be coated or otherwise treated so as to be rendered hydrophobic. In some embodiments, the barrels 20 has a hydrophobic interior wall characterized by the absence of a lubricant such as, but not limited to, silicone or silicone oil. As used herein, the term "hydrophobic interior wall" refers to the interior surface of a barrel that is free or substantially free (i.e., has an unquantifiable or trace amount) of silicone oil. In addition, the hydrophobic surface of the barrel 20 also has a contact angle of deionized water on a flat surface of the material greater than 90°, indicating a hydrophobic surface. In some embodiments, the water contact angle is from about 90° to about 180° or from about 96° to about 180°, from about 96° to about 130, or from about 96° to about 120°.

In some embodiments, the body 240 of the stopper 40 is formed of a suitable elastomer, such as a rubber material. Examples of suitable rubber materials include synthetic rubbers, thermoplastic elastomers, and materials prepared by blending synthetic rubbers and the thermoplastic elastomers. The material may be rubbers constructed from butyl, bromobutyl, or chlorobutyl, a halogenated butyl rubber, a styrene butadiene rubber, a butadiene rubber, an epichlorohydrin rubber, a neoprene rubber, an ethylene propylene rubber, silicone, nitrile, styrene butadiene, polychloroprene, ethylene propylene diene, fluoroelastomers, thermoplastic elastomers (TPE), thermoplastic vulcanizates (TPV), materials sold under the trade name VITON^{®}, and combinations and blends thereof. In some embodiments, the body 240 may have an initial modulus (small strain) of between about 2.5 MPa to about 5 MPa, or between about 3 MPa to about 4 MPa. In some embodiments, the initial modulus is about 3.5 MPa, although a variety of values are contemplated.

As previously referenced, portions of the barrier 242 (e.g., layers or zones) may be configured to be more activatable, or reactive, to an energy source than other layers or zones of the barrier 242. For example, in the case of laser or other optical energy sources, the reactivity or ability to be activated, may be adjusted by modifying material thickness, pigmentation, density/open space/air content, chemical / material composition, and others. In the case of radiofrequency (RF), electrical and electromagnetic energy sources, the barrier 242 may be adjusted to include pigments or other fillers, such as metallics (e.g., iron, platinum, or others), that are more reactive to such energy. In the case of microwave energy sources, metallics, water, or other materials may be implemented. And, in the case of ultraviolet (UV) energy cross-linking agents (acrylates that would cross-link and increase density / stiffness) or other materials that absorb UV energy may be incorporated.

Examples suitable materials for one or more layers of the barrier 242 of the stopper include films of ultrahigh molecular weight polyethylenes and fluororesins. The barrier 242 may include a fluoropolymer film, such as a polytetrafluoroethylene (PTFE) film or a densified expanded polytetrafluoroethylene (ePTFE) film. Film and film composites including PTFE or ePTFE can help provide thin and strong barrier layers to leachables and extractables that may be present in the underlying elastomer and might otherwise contaminate the therapeutic liquid in the barrel.

Some specific examples of suitable materials of the barrier 242 include, but are not limited to, the following: (1) A PTFE (polytetrafluoroethylene) homopolymer film produced by the skiving method (e.g., VALFLON (trade name) available from Nippon Valqua Industries, Ltd.); (2) A modified PTFE (a copolymer of a tetrafluoroethylene monomer and several percents of a perfluoroalkoxide monomer) film produced by the skiving method (e.g., NEW VALFLON (trade name) available from Nippon Valqua Industries, Ltd.); and (3) An ultrahigh molecular weight polyethylene film produced by the skiving method (e.g., NEW LIGHT NL-W (trade name) available from Saxin Corporation).

As indicated, the barrier 242 may be a composite or laminate material, or otherwise include a multi-component (e.g., multi-layer) barrier. Other suitable fluoropolymers for use in or as the barrier 242 include, but are not limited to, fluorinated ethylene propylene (FEP), polyvinylidene fluoride, polyvinylfluoride, perfluoropropylvinylether, perfluoroalkoxy polymers, tetrafluoroethylene (TFE), Parylene AF-4, Parylene VT-4, and copolymers and combinations thereof. Non-fluoropolymers such as, but not limited to, polyethylene, polypropylene, Parylene C, and Parylene N may also or alternatively be used to form the barrier 242.

A densified ePTFE film for the barrier 242 may be prepared in the manner described in U.S. Pat. 7,521,010 to Kennedy, et al., U.S. Pat. No. 6,030,694 to Dolan et al., U.S. Pat. No. 5,792,525 to Fuhr et al., or U.S. Pat. No. 5,374,473 to Knox et al. Expanded copolymers of PTFE may also be used for the barrier 242, such as those described in U.S. Pat. No. 5,708,044 to Branca, U.S. Pat. No. 6,541,589 to Baillie, U.S. Pat. No. 7,531,611 to Sabol et al., U.S. Pat. No. 8,637,144 to Ford, and U.S. Pat. No. 9,139,669 to Xu et al., particularly if they are densified.

In one or more embodiment, the barrier 242 may include, or be formed of, one or more of the following materials: ultra-high molecular weight polyethylene as taught in U.S. Pat. No. 9,926,416 to Sbriglia; polyparaxylylene as taught in U.S. Patent Publication No. 2016/0032069 to Sbriglia; polylactic acid as taught in U.S. Pat. No. 9,732,184 to Sbriglia, et al.; and/or VDF-co-(TFE or TrFE) polymers as taught in U.S. Pat. No. 9,441,088 to Sbriglia.

The barrier 242 may also include an expanded polymeric material including a functional tetrafluoroethylene (TFE) copolymer material having a microstructure characterized by nodes interconnected by fibrils, where the functional TFE copolymer material includes a functional copolymer of TFE and PSVE (perfluorosulfonyl vinyl ether), or TFE with another suitable functional monomer, such as, but not limited to, vinylidene fluoride (VDF), vinyl acetate, or vinyl alcohol. The functional TFE copolymer material may be prepared, for example, according to the methods described in U.S. Pat. No. 9,139,669 to Xu et al. or U.S. Pat. No. 8,658,707 to Xu et al.

In some embodiments, the barrier 242 may be formed of a composite fluoropolymer or non-fluoropolymer material having a barrier layer and a tie layer such as is described in U.S. Patent Publication No. 2016/0022918 to Gunzel. It is to be noted that, as used herein, the term "tie layer" may include fluoropolymer and/or non-fluoropolymer materials. The tie layer can include, or be formed of, expanded polytetrafluoroethylene or other porous expanded fluoropolymers (for example, an ePTFE as taught in U.S. Pat. No. 6,541,589 to Baille). Alternatively, the tie layer may be formed of, or include, non-fluoropolymer materials. Non-limiting examples of suitable non-fluoropolymer materials for use in or as the tie layer include non-fluoropolymer membranes, non-fluoropolymer microporous membranes, non-woven materials (e.g., spunbonded, melt blown fibrous materials, electrospun nanofibers), polyvinylidene difluoride (PVDF), nanofibers, polysulfones, polyethersulfones, polyarlysolfones, polyether ether ketone (PEEK), polyethylenes, polypropylenes, and polyimides.

In some embodiments, the barrier 242 can be made by forming a thin densified composite comprising a porous ePTFE layer and a thermoplastic barrier layer. In this aspect, a thermoplastic having a surface with a low coefficient of friction is preferred. Accordingly, fluoropolymer-based thermoplastics such as fluorinated ethylene propylene (FEP), perfluoroalkoxy (PFA), a polymer of tetrafluoroethylenes, hexafluoropropylene and vinylindene fluoride (THV) may be applicable. A barrier according to this aspect may be an FEP/ePTFE laminate obtained by following the process taught in WO 94/13469 to Bacino. The barrier may be formed at process temperatures above the softening temperature or even above the melt of the FEP film in a female cavity mold.

In some embodiments, the barrier 242 may comprise a composite of a densified ePTFE film and a thin layer of porous ePTFE bonded to the barrier layer film. The densified ePTFE film may be obtained as described in U.S. Pat. No. 7,521,010 to Kennedy et al. The ePTFE/densified ePTFE composite may be combined in the manner described in U.S. Pat. No. 6,030,694 to Dolan, et al. In this embodiment, the composite material comprises a layer of densified ePTFE film and a porous ePTFE layer.

In some embodiments, the barrier 242 includes a composite material having at least three layers, namely, a densified expanded fluoropolymer layer, a barrier melt fluoropolymer layer, and a porous layer. The densified expanded fluoropolymer layer may include or be formed of a densified ePTFE. The barrier melt fluoropolymer layer may include a fluoropolymer such as a densified expanded fluoropolymer, polytetrafluoroethylene (PTFE), expanded polytetrafluorethylene (ePTFE), densified expanded polytetrafluoroethylene, fluorinated ethylene propylene (FEP), polyvinylidene fluoride, polyvinylfluoride, perfluoropropylvinylether, perfluoroalkoxy polymers, and copolymers and combinations thereof. Non-limiting examples of non-fluoropolymers that may be utilized in the barrier melt layer include polyethylene and polypropylene. The porous layer may include or be formed of ePTFE or other porous expanded fluoropolymers. The laminate layers having the densified expanded fluoropolymer layer, the barrier melt fluoropolymer layer and the porous layer 180 may be constructed by coating or otherwise depositing the densified expanded fluoropolymer onto the porous layer to create the composite material. In one non-limiting embodiment, the laminate layer 130 is formed of a densified fluoropolymer (e.g., densified ePTFE), a thermoplastic adhesive (e.g., FEP), and a porous fluoropolymer (e.g., ePTFE).

It is to be appreciated that the stopper 40 may include various degrees of penetration of either the material of the body 240 into the materials of the barrier 242 or vice versa, including those described in U.S. Pat. No. 8,722,178 to Ashmead, et al., U.S. Pat. No. 9,597,458 to Ashmead, et al., and U.S. Patent Publication No. 2016/0022918 to Gunzel. It is also to be appreciated that there are many variations of the processes described herein that could be utilized for forming the stopper 40 without departing from the scope of the invention.

### Examples of Therapeutic Substances

The syringes, tip caps, and other embodiments of the present disclosure may be used in combination with different therapeutic compounds including, but not limited to, drugs and biologics such as Coagulation Factors, Cytokines, Epigenetic protein families, Growth Factors, Hormones, Peptides, Signal Transduction molecules, and mutations thereof; also including Amino Acids, Vaccines and/or combinations thereof. Therapeutic compounds further include antibodies, antisense, RNA interference made to the above biologics and their target receptors and mutations of thereof. Additional therapeutic compounds include Gene Therapy, Primary and Embryonic Stem Cells. Also included in the therapeutic compounds are antibodies, antisense, RNA interference to Protein Kinases, Esterases, Phosphatases, Ion channels, Proteases, structural proteins, membrane transport proteins, nuclear hormone receptors and/or combinations thereof. Additionally, it is to be understood that at least one of the therapeutic compounds identified herein used in the instant disclosure, also two or more therapeutic compounds listed in this application are considered to be within the purview of the present disclosure.

Examples of Coagulation Factors include, but are not limited to: Fibrinogen, Prothrombin, Factor I, Factor V, Factor X, Factor VII, Factor VIII, Factor XI, Factor XIII, Protein C, Platelets, Thromboplastin, and Co-factor of VIIa.

Examples of Cytokines include, but are not limited to: Lymphokines, Interleukins, Chemokines, Monokines, Interferons, and Colony stimulating factors.

Examples of Epigenetic protein families include, but are not limited to: ATPase family AAA domain-containing protein 2 (ATAD2A), ATPase family-AAA domain containing 2B (ATAD2B), ATPase family AAA domain containing-2B (ATAD2B), bromodomain adjacent to zinc finger domain-1A (BAZ1A), bromodomain adjacent to zinc finger domain-1B (BAZ1B), bromodomain adjacent to zinc finger domain-2A (BAZ2A), bromodomain adjacent to zinc finger domain-2A (BAZ2A), bromodomain adjacent to zinc finger domain-2B (BAZ2B), bromodomain-containing protein 1 (BRD1), Bromodomain containing protein 2-1st bromodomain (BRD2), Bromodomain containing protein 2-1st & 2nd bromodomains (BRD2), bromodomain-containing protein 2 isoform 1-bromodomain 2 (BRD2(2)), bromodomain-containing protein 3-bromodomain 1 (BRD3(1)), Bromodomain-containing protein 3-1st bromodomain (BRD3), Bromodomain-containing protein 3-1st & 2nd bromodomains (BRD3), bromodomain-containing protein 3-bromodomain 2 (BRD3(2)), Bromodomain containing protein 4-1st bromodomain (BRD4), bromodomain-containing protein 4 isoform long-bromodomains 1 and 2 (BRD4(1-2)), bromodomain-containing protein 4 isoform long-bromodomain 2 (BRD4(2)), bromodomain-containing protein 4 isoform short (BRD4(full-length-short-iso.)), Bromodomain containing protein 7 (BRD7), bromodomain containing 8-bromodomain 1 (BRD8(1)), bromodomain containing 8-bromodomain 2 (BRD8(2)), bromodomain-containing protein 9 isoform 1 (BRD9), Bromodomain containing testis-specific-1st bromodomain (BRDT), Bromodomain containing testis-specific-1st & 2nd bromodomains (BRDT), bromodomain testis-specific protein isoform b-bromodomain 2 (BRDT(2)), bromodomain and PHD finger containing-1 (BRPF1), bromodomain and PHD finger containing-3 (BRPF3), bromodomain and PHD finger containing-3 (BRPF3), Bromodomain and WD repeat-containing 3-2nd bromodomain (BRWD3(2)), Cat eye syndrome critical region protein 2 (CECR2), CREB binding protein (CREBBP), E1A binding protein p300 (EP300), EP300 (EP300), nucleosome-remodeling factor subunit BPTF isoform 1 (FALZ), Nucleosome-remodeling factor subunit BPT (FALZ), Euchromatic histone-lysine N-methyltransferase 2 (EHMT2), Histone Acetyltransferase-KAT2A (GCN5L2), Euchromatic histone-lysine N-methyltransferase 1 (EHMT1), Histone-lysine N-methyltransferase MLL (MLL), Polybromo 1-1 st bromodomain (PB1(1)), Polybromo 1-2nd bromodomain (PB1(2)), polybromo 1-bromodomain 2 (PBRM1(2)), polybromo 1-bromodomain 5 (PBRM1(5)), Histone acetyltransferase KAT2B (PCAF), PH-interacting protein-1st bromodomain (PHIP(1)), PH-interacting protein-2nd bromodomain (PHIP(2)), Protein kinase C-binding protein 1 (PRKCBP1), Protein arginine N-methyltransferase 3 (PRMT3), SWI/SNF related-matrix associated-actin dependent regulator of chromatin-subfamily a-member 2 (SMARCA2), SWI/SNF related-matrix associated-actin dependent regulator of chromatin-subfamily a-member 4 (SMARCA4), Nuclear body protein-SP110 (SP110), Nuclear body protein-SP140 (SP140), Transcription initiation factor TFIID subunit 1 (TAF1(1-2)), TAF1 RNA polymerase II-TATA box binding protein (TBP)-associated factor-250 kDa-bromodomain 2 (TAF1(2)), Transcription initiation factor TFIID subunit 1-like-1 st bromodomain (TAF1L(1)), Transcription initiation factor TFIID subunit 1-like-2nd bromodomain (TAF1L(2)), tripartite motif containing 24 (TRIM24(Bromo.)), tripartite motif containing 24 (TRIM24(PHD-Bromo.)), E3 ubiquitin-protein ligase TRIM33 (TRIM33), tripartite motif containing 33 (TRIM33(PHD-Bromo.)), WD repeat 9-1st bromodomain (WDR9(1)), and WD repeat 9-2nd bromodomain (WDR9(2)).

Examples of growth factors include, but are not limited to: nerve growth factor (NGF), vascular endothelial growth factor (VEGF), platelet-derived growth factor (PDGF), C-fos-induced growth factor (FIGF), platelet-activating factor (PAF), transforming growth factor beta (TGF-β), bone morphogenetic proteins (BMPs), Activin, inhibin, fibroblast growth factors (FGFs), granulocyte-colony stimulating factor (G-CSF), granulocyte-macrophage colony stimulating factor (GM-CSF), glial cell line-derived neurotrophic factor (GDNF), growth differentiation factor-9 (GDF9), epidermal growth factor (EGF), transforming growth factor-α (TGF-α), growth factor (KGF), migration-stimulating factor (MSF), hepatocyte growth factor-like protein (HGFLP), hepatocyte growth factor (HGF), hepatoma-derived growth factor (HDGF), and Insulin-like growth factors.

Examples of Hormones include, but are not limited to: Amino acid derived (such as melatonin and thyroxine), Thyrotropin-releasing hormone, Vasopressin, Insulin, Growth Hormones, Glycoprotein Hormones, Luteinizing Hormone, Follicle-stimulating Hormone, Thyroid-stimulating hormone, Eicosanoids, Arachidonic acid, Lipoxins, Prostaglandins, Steroid, Estrogens, Testosterone, Cortisol, and Progestogens.

Examples of Proteins and Peptides and Signal Transduction molecules include, but are not limited to: Ataxia Telangiectasia Mutated, Tumor Protein p53, Checkpoint kinase 2, breast cancer susceptibility protein, Double-strand break repair protein, DNA repair protein RAD50, Nibrin, p53-binding protein, Mediator of DNA damage checkpoint protein, H2A histone family member X, Microcephalin, C-terminal-binding protein 1, Structural maintenance of chromosomes protein 1A, Cell division cycle 25 homolog A (CDC25A), forkhead box O3 (forkhead box O3), nuclear factor of kappa light polypeptide gene enhancer in B-cells inhibitor, alpha (NFKBIA), nuclear factor (erythroid-derived 2)-like 2 (NFE2L2), Natriuretic peptide receptor A (NPR1), Tumor necrosis factor receptor superfamily, member 11a (TNFRSF11A), v-rel reticuloendotheliosis viral oncogene homolog A (avian) (RELA), Sterol regulatory element binding transcription factor 2 (SREBF2), CREB regulated transcription coactivator 1 (CRTC1), CREB regulated transcription coactivator 2 (CRTC2), X-box binding protein 1 (XBP1), and Catenin beta 1 (cadherin-associated protein or CTNNB1).

Examples of G Protein-Coupled Receptors (GPCR) include, but are not limited to: Adenosine receptor family, Adrenergic receptor family, Angiotensin II receptor, Apelin receptor, Vasopressin receptor family, Brain-specific angiogenesis inhibitor family, Bradykinin receptor family, Bombesin receptor family, Complement component 3a receptor 1, Complement component 5a receptor 1, Calcitonin receptor family, Calcitonin receptor-like family, Calcium-sensing receptor, Cholecystokinin A receptor (CCK1), Cholecystokinin B receptor (CCK2), Chemokine (C-C motif) receptor family, Sphingosine 1-phosphate receptor family, Succinic receptor, Cholinergic receptor family. Chemokine-like receptor family, Cannabinoid receptor family, Corticotropin releasing hormone receptor family, prostaglandin D2 receptor, Chemokine C-X3-C receptor family, Chemokine (C-X-C motif) receptor family, Burkitt lymphoma receptor, Chemokine (C-X-C motif) receptor family, Cysteinyl leukotriene receptor 2 (CYSLT2), chemokine receptor (FY), Dopamine receptor family, G protein-coupled receptor 183 (GPR183), Lysophosphatidic acid receptor family, Endothelin receptor family, Coagulation factor II (thrombin) receptor family, Free fatty acid receptor family, Formylpeptide receptor family, Follicle stimulating hormone receptor (FSHR), gamma-aminobutyric acid (GABA) B receptor, Galanin receptor family, Glucagon receptor, Growth hormone releasing hormone receptor (GHRH), Ghrelin receptor (ghrelin), Growth hormone secretagogue receptor 1b (GHSR1b), Gastric inhibitory polypeptide receptor (GIP), Glucagon-like peptide receptor family, Gonadotropin-releasing hormone receptor (GnRH), pyroglutamylated RFamide peptide receptor (QRFPR), G protein-coupled bile acid receptor 1 (GPBA), Hydroxycarboxylic acid receptor family, Lysophosphatidic acid receptor 4 (LPA4) Lysophosphatidic acid receptor 5 (GPR92), G protein-coupled receptor 79 pseudogene (GPR79), Hydroxycarboxylic acid receptor 1 (HCA1), G-protein coupled receptor (C5L2, FFA4, FFA4, FFA4, GPER, GPR1, GPR101, GPR107, GPR119, GPR12, GPR123, GPR132, GPR135, GPR139, GPR141, GPR142, GPR143, GPR146, GPR148, GPR149, GPR15, GPR150, GPR151, GPR152, GPR157, GPR161, GPR162, GPR17, GPR171, GPR173, GPR176, GPR18, GPR182, GPR20, GPR22, GPR25, GPR26, GPR27, GPR3, GPR31, GPR32, GPR35, GPR37L1, GPR39, GPR4, GPR45, GPR50, GPR52, GPR55, GPR6, GPR61, GPR65, GPR75, GPR78, GPR83, GPR84, GPR85, GPR88, GPR97, TM7SF1), Metabotropic glutamate receptor family, Gastrin releasing peptide receptor (BB2), Orexin receptor family, Histamine receptor family, 5-hydroxytryptamine receptor family, KISS1-derived peptide receptor (kisspeptin), Leucine-rich repeat-containing G protein-coupled receptor family, horiogonadotropin receptor (LH), Leukotriene B4 receptor (BLT1), Adenylate Cyclase Activating Polypeptide 1 Receptor 1 (mPAC1), Motilin receptor, Melanocortin receptor family, Melanin concentrating hormone receptor 1 (MCH1), Neuropeptide Y1 receptor (Y1), Neuropeptide Y2 receptor (NPY2R), Opioid receptor family, Oxytocin receptor (OT), P2Y Purinoceptor 12 (mP2Y12), P2Y Purinoceptor 6 (P2Y6), Pancreatic polypeptide receptor family, Platelet-activating factor receptor family, Prostaglandin E receptor family, Prostanoid IP1 receptor (IP1), MAS-related GPR, member family, Rhodopsin (Rhodopsin), Relaxin family peptide receptor family, Somatostatin receptor family, Tachykinin receptor family, Melatonin receptor family, Urotensin receptor family, Vasoactive intestinal peptide receptor 1 (mVPAC1), Neuromedin B Receptor (BB1), Neuromedin U receptor 1 (NMU1), Neuropeptides B/W receptor family, Neuropeptide FF receptor 1 (NPFF1), neuropeptide S receptor 1 (NPS receptor), Neuropeptide Y receptor family, Neurotensin receptor 1 (NTS1), Opsin 5 (OPN5), Opioid receptor-like receptor (NOP), Oxoeicosanoid (OXE) receptor 1 (OXE), Oxoglutarate (alpha-ketoglutarate) receptor 1 (OXGR1), Purinergic receptor family, Pyrimidinergic receptor family, Prolactin releasing hormone receptor (PRRP), Prokineticin receptor family, Platelet activating receptor (PAF), Prostaglandin F receptor family, Prostaglandin 12 (prostacyclin) receptor family, Parathyroid hormone receptor family, muscarinic acetylcholine receptors (such as rM4), Prostanoid DP2 receptor (rGPR44), Prokineticin receptor family, Relaxin family peptide receptor family, Secretin receptor (secretin), Frizzled class receptor (Smoothened), trace amine associated receptor family, Tachykinin family, Thromboxane A2 receptor (TP), Thyrotropin-releasing hormone receptor (TRH1), and Thyroid Stimulating Hormone Receptor (TSH).

Examples of nuclear hormone receptors include, but are not limited to: Androgen receptor (AR), Estrogen related receptor alpha (ESRRA), Estrogen receptor 1 (ESR1), Nuclear receptor subfamily 1-group H-member 4 (NR1H4), Nuclear receptor subfamily 3-group C-member 1 (glucocorticoid receptor) (NR3C1), Nuclear receptor subfamily 1-group H-member 3 (Liver X receptor α) (NR1H3), Nuclear receptor subfamily 1-group H-member 2 (Liver X receptor β) (NR1H2), Nuclear receptor subfamily 1-group H-member 2 (Liver X receptor β) (NR1H2), Nuclear receptor subfamily 3-group C-member 2 (Mineralocorticoid receptor) (NR3C2), Peroxisome Proliferator Activated Receptor alpha (PPARA), Peroxisome Proliferator Activated Receptor gamma (PPARG), Peroxisome Proliferator Activated Receptor delta (PPARD), Progesterone receptor α (PGR), Progesterone receptor β (PGR), Retinoic acid receptor-alpha (RARA), Retinoic acid receptor-beta (RARB), Retinoid X receptor-alpha (RXRA), Retinoid X receptor-gamma (RXRG), Thyroid hormone receptor-alpha (THRA), Thyroid hormone receptor-beta (THRB), Retinoic acid-related orphan receptor, Liver X receptor, Farnesoid X receptor, Vitamin D receptor, Pregnane X receptor, Constitutive androstane receptor, Hepatocyte nuclear factor 4, Oestrogen receptor, Oestrogen-related receptor, Glucocortioic receptor, and Nerve growth factor-induced-B, Germ cell nuclear factor.

Examples of membrane transport proteins include, but are not limited to: ATP-binding cassette (ABC) superfamily, solute carrier (SLC) superfamily, multidrug resistance protein 1 (P-glycoprotein), organic anion transporter 1, and proteins such as EAAT3, EAAC1, EAAT1, GLUT1, GLUT2, GLUT9, GLUT10, rBAT, AE1, NBC1, KNBC, CHED2, BTR1, NABC1, CDPD, SGLT1, SGLT2, NIS, CHT1, NET, DAT, GLYT2, CRTR, BOAT1, SIT1, XT3, y+LAT1, BAT1, NHERF1, NHE6, ASBT, DMT1, DCT1, NRAMP2, NKCC2, NCC, KCC3, NACT, MCT1, MCT8, MCT12, SLD, VGLUT3, THTR1, THTR2, PIT2, GLVR2, OCTN2, URAT1, NCKX1, NCKX5, CIC, PiC, ANTI, ORNT1, AGC1, ARALAR, Citrin, STLN2, aralar2, TPC, MUP1, MCPHA, CACT, GC1, PHC, DTD, CLD, DRA, PDS, Prestin, TAT1, FATP4, ENT3, ZnT2, ZnT10, AT1, NPT2A, NPT2B, HHRH, CST, CDG2F, UGAT, UGTL, UGALT, UGT1, UGT2, FUCT1, CDG2C, NST, PAT2, G6PT1, SPX4, ZIP4, LIV4, ZIP13, LZT-Hs9, FPN1, MTP1, IREG1, RHAG, AIM1, PCFT, FLVCR1, FLVCR2, RFT1, RFT2, RFT3, OATP1B1, OATP1B3, and OATP2A1.

Examples of structural proteins include, but are not limited to: tubulin, heat shock protein, Microtubule-stabilizing proteins, Oncoprotein 18, stathmin, kinesin-8 and kinesin-14 family, Kip3, and Kif18A.

Examples of proteases include, but are not limited to ADAM (a disintegrin and metalloprotease) family.

Examples of Protein kinases include, but are not limited to: AP2 associated kinase, Homo sapiens ABL proto-oncogene 1-non-receptor tyrosine-protein kinase family, c-abl oncogene 1 receptor tyrosine kinase family, v-abl Abelson murine leukemia viral oncogene homolog 2, activin A receptor family, chaperone-ABC1 activity of bc1 complex homolog (S. pombe) (ADCK3), aarF domain containing kinase 4 (ADCK4), v-akt murine thymoma viral oncogene homolog family, anaplastic lymphoma receptor tyrosine kinase family, protein kinase A family, protein kinase B family, ankyrin repeat and kinase domain containing 1 (ANKK1), NUAK family-SNF1-like kinase, mitogen-activated protein kinase kinase kinase family aurora kinase A (AURKA), aurora kinase B (AURKB), aurora kinase C (AURKC), AXL receptor tyrosine kinase (AXL), BMP2 inducible kinase (BIKE), B lymphoid tyrosine kinase (BLK), bone morphogenetic protein receptor family, BMX non-receptor tyrosine kinase (BMX), v-raf murine sarcoma viral oncogene homolog B1 (BRAF), protein tyrosine kinase 6 (BRK), BR serine/threonine kinase family, Bruton agammaglobulinemia tyrosine kinase (BTK), calcium/calmodulin-dependent protein kinase family, cyclin-dependent kinase family, cyclin-dependent kinase-like family, CHK1 checkpoint homolog (S. pombe) (CHEK1), CHK2 checkpoint homolog (S. pombe) (CHEK2), Insulin receptor, isoform A (INSR), Insulin receptor, isoform B (INSR), rho-interacting serine/threonine kinase (CIT), v-kit Hardy-Zuckerman 4 feline sarcoma viral oncogene homolog (KIT), CDC-Like Kinase family-Hepatocyte growth factor receptor (MET), Proto-oncogene tyrosine-protein kinase receptor, colony-stimulating factor family receptor, c-src tyrosine kinase (CSK), casein kinase family, megakaryocyte-associated tyrosine kinase (CTK), death-associated protein kinase family, doublecortin-like kinase family, discoidin domain receptor tyrosine kinase, dystrophia myotonica-protein kinase (DMPK), dual-specificity tyrosine-(Y)-phosphorylation regulated kinase family, epidermal growth factor receptor family, eukaryotic translation initiation factor 2-alpha kinase 1 (EIF2AK1), EPH receptor family, Ephrin type-A receptor family, Ephrin type-B receptor family, v-erb-b2 erythroblastic leukemia viral oncogene homolog family, mitogen-activated protein kinase family, endoplasmic reticulum to nucleus signaling 1 (ERN1), PTK2 protein tyrosine kinase 2 (FAK), fer (fps/fes related) tyrosine kinase (FER). feline sarcoma oncogene (FES), Fibroblast growth factor receptor family, Gardner-Rasheed feline sarcoma viral (v-fgr) oncogene homolog (FGR), fms-related tyrosine kinase family, Fms-related tyrosine kinase family, fyn-related kinase (FRK), FYN oncogene related to SRC, cyclin G associated kinase (GAK), eukaryotic translation initiation factor 2 alpha kinase, Growth hormone receptor. G protein-coupled receptor kinase 1 (GRK1), G protein-coupled receptor kinase family, glycogen synthase kinase family, germ cell associated 2 (haspin) (HASPIN), Hemopoietic cell kinase (HCK), homeodomain interacting protein kinase family, mitogen-activated protein kinase kinase kinase kinase family, hormonally up-regulated Neu-associated kinase (HUNK), intestinal cell (MAK-like) kinase (ICK), Insulin-like growth factor 1 receptor (IGF1R), conserved helix-loop-helix ubiquitous kinase (IKK-alpha), inhibitor of kappa light polypeptide gene enhancer in B-cells-kinase beta family, insulin receptor (INSR), insulin receptor-related receptor (INSRR), interleukin-1 receptor-associated kinase family, IL2-inducible T-cell kinase (ITK), Janus kinase family, Kinase Insert Domain Receptor, v-kit Hardy-Zuckerman 4 feline sarcoma viral oncogene homolog, lymphocyte-specific protein tyrosine kinase (LCK), LIM domain kinase family, serine/threonine kinase family leucine-rich repeat kinase family, v-yes-1 Yamaguchi sarcoma viral related oncogene homolog (LYN), male germ cell-associated kinase (MAK); MAP/microtubule affinity-regulating kinase family such as microtubule associated serine/threonine kinase family, maternal embryonic leucine zipper kinase, c-mer proto-oncogene tyrosine kinase (MERTK), met proto-oncogene (hepatocyte growth factor receptor), MAP kinase interacting serine/threonine kinase family, myosin light chain kinase family, mixed lineage kinase domain-like protein isoform, CDC42 binding protein kinase family, serine/threonine kinase family, macrophage stimulating 1 receptor (c-met-related tyrosine kinase) (MST1R), mechanistic target of rapamycin (serine/threonine kinase) (MTOR), muscle-skeletal-receptor tyrosine kinase (MUSK), myosin light chain kinase family, NIMA (never in mitosis gene a)-related kinase family, serine/threonine-protein kinase NIM1 (NIM1), nemo-like kinase (NLK), oxidative-stress responsive 1 (OSR1), p21 protein (Cdc42/Rac)-activated kinase family, PAS domain containing serine/threonine kinase, Platelet-derived growth factor receptor family, 3-phosphoinositide dependent protein kinase-1 (PDPK1), Calcium-dependent protein kinase 1, phosphorylase kinase gamma family, Phosphatidylinositol 4,5-bisphosphate 3-kinase, phosphoinositide-3-kinase family, phosphatidylinositol 4-kinase family. phosphoinositide kinase, FYVE finger containing, Pim-1 oncogene (PIM1), pim-2 oncogene (PIM2), pim-3 oncogene (PIM3), phosphatidylinositol-4-phosphate 5-kinase family, phosphatidylinositol-5-phosphate 4-kinase family protein kinase, membrane associated tyrosine/threonine 1 (PKMYT1), protein kinase N family, polo-like kinase family, protein kinase C family, protein kinase D family, cGMP-dependent protein kinase family, eukaryotic translation initiation factor 2-alpha kinase 2 (PRKR), X-linked protein kinase (PRKX), Prolactin receptor (PRLR), PRP4 pre-mRNA processing factor 4 homolog B (yeast) (PRP4), PTK2B protein tyrosine kinase 2 beta (PTK2B), SIK family kinase 3 (QSK), v-raf-1 murine leukemia viral oncogene homolog 1 (RAF1), Neurotrophic tyrosine kinase receptor type family, receptor (TNFRSF)-interacting serine-threonine kinase family, dual serine/threonine and tyrosine protein kinase (RIPK5), Rho-associated, coiled-coil containing protein kinase family, c-ros oncogene 1, receptor tyrosine kinase (ROS1), ribosomal protein S6 kinase family, SH3-binding domain kinase 1 (SBK1), serum/glucocorticoid regulated kinase family, Putative uncharacterized serine/threonine-protein kinase (Sugen kinase 110) (SgK110), salt-inducible kinase family, SNF related kinase (SNRK), src-related kinase, SFRS protein kinase family; Spleen tyrosine kinase (SYK) such as TAO kinase family; TANK-binding kinase 1 (TBK1) such as tec protein tyrosine kinase (TEC), testis-specific kinase 1 (TESK1), transforming growth factor, beta receptor family, tyrosine kinase with immunoglobulin-like and EGF-like domains 1 (TIE1), TEK tyrosine kinase, endothelial (TIE2), Angiopoietin-1 receptor (Tie2), tousled-like kinase family, TRAF2 and NCK interacting kinase (TN IK), non-receptor tyrosine kinase family, TNNI3 interacting kinase (TNNI3K), transient receptor potential cation channel, testis-specific serine kinase family, TTK protein kinase (TTK), TXK tyrosine kinase (TXK), Tyrosine kinase 2 (TYK2), TYRO3 protein tyrosine kinase (TYRO3), unc-51-like kinase family, phosphatidylinositol 3-kinase, vaccinia related kinase 2 (VRK2), WEE1 homolog family, WNK lysine deficient protein kinase family, v-yes-1 Yamaguchi sarcoma viral oncogene homolog 1 (YES), sterile alpha motif and leucine zipper containing kinase AZK (ZAK), and zeta-chain (TCR) associated protein kinase 70 kDa (ZAP70).

Cell therapy using cells that are derived primarily from: endoderm such as Exocrine secretory epithelial cells and Hormone-secreting cells; ectoderm such as Keratinizing epithelial cells, Wet stratified barrier epithelial cells, Sensory transducer cells, Autonomic neuron cells, Sense organ and peripheral neuron supporting cells, Central nervous system neurons and glial cells, Lens cells; mesoderm such as Metabolism and storage cells, Barrier function cells (lung, gut, exocrine glands and urogenital tract), Extracellular matrix cells, Contractile cells, Blood and immune system cells, Germ cells, Nurse cell, Interstitial cells and combinations thereof. Additionally, in the scope of the invention are cells that are genetically, chemically or physically altered or otherwise modified.

Examples of Exocrine secretory epithelial cells include but are not limited to: Salivary gland mucous cell, Salivary gland number 1, Von Ebner's gland cell in tongue, Mammary gland cell, Lacrimal gland cell, Ceruminous gland cell in ear, Eccrine sweat gland dark cell, Eccrine sweat gland clear cell, Apocrine sweat gland cell, Gland of Moll cell in eyelid, Sebaceous gland cell, Bowman's gland cell in nose, Brunner's gland cell in duodenum, Seminal vesicle cell, Prostate gland cell, Bulbourethral gland cell, Bartholin's gland cell, Gland of Littre cell, Uterus endometrium cell, Isolated goblet cell of respiratory and digestive tracts, Stomach lining mucous cell, Gastric gland zymogenic cell, Gastric gland oxyntic cell, Pancreatic acinar cell, Paneth cell of small intestine, Type II pneumocyte of lung, and Clara cell of lung; Hormone-secreting cells including, but not limited to: Anterior pituitary cells, Intermediate pituitary cell, Magnocellular neurosecretory cells, Gut and respiratory tract cells, Thyroid gland cells, Parathyroid gland cells, Adrenal gland cells, Leydig cell of testes secreting testosterone, Theca interna cell of ovarian follicle secreting estrogen, Corpus luteum cell of ruptured ovarian follicle secreting progesterone, Juxtaglomerular cell, Macula densa cell of kidney, Peripolar cell of kidney, Mesangial cell of kidney, and Pancreatic islets; Keratinizing epithelial cells including, but not limited to: Epidermal keratinocyte, Epidermal basal cell, Keratinocyte of fingernails and toenails, Nail bed basal cell, Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, and Hair matrix cell; Wet stratified barrier epithelial cells including, but not limited to: Surface epithelial cell of stratified squamous epithelium and basal cell of epithelia of cornea, tongue, oral cavity, esophagus, anal canal, distal urethra and vagina, and Urinary epithelium cell; Sensory transducer cells including, but not limited to: Auditory inner hair cell of organ of Corti, Auditory outer hair cell of organ of Corti, Basal cell of olfactory epithelium, Cold-sensitive primary sensory neurons, Heat-sensitive primary sensory neurons, Merkel cell of epidermis, Olfactory receptor neuron, Pain-sensitive primary sensory neurons, Photoreceptor cells of retina in eye, Proprioceptive primary sensory neurons, Touch-sensitive primary sensory neurons, Type I carotid body cell, Type II carotid body cell, Type I hair cell of vestibular system of ear, Type II hair cell of vestibular system of ear, and Type I taste bud cell; Autonomic neuron cells including, but not limited to: Cholinergic neural cell, Adrenergic neural cell, and Peptidergic neural cell; Sense organ and peripheral neuron supporting cells including, but not limited to: Inner pillar cell of organ of Corti, Outer pillar cell of organ of Corti, Inner phalangeal cell of organ of Corti, Outer phalangeal cell of organ of Corti, Border cell of organ of Corti, Hensen cell of organ of Corti, Vestibular apparatus supporting cell, Taste bud supporting cell, Olfactory epithelium supporting cell, Schwann cell, Satellite glial cell, and Enteric glial cell; Central nervous system neurons and glial cells including, but not limited to: Astrocyte, Neuron cells, Oligodendrocyte, and Spindle neuron; Lens cells including, but not limited to: Anterior lens epithelial cell, and Crystallin-containing lens fiber cell; Metabolism and storage cells including, but not limited to: Adipocytes, and Liver lipocyte; Barrier function cells including, but not limited to: Kidney parietal cell, Kidney glomerulus podocyte, Kidney proximal tubule brush border cell, Loop of Henle thin segment cell, Kidney distal tubule cell, Kidney collecting duct cell, Principal cells, Intercalated cells, Type I pneumocyte, Pancreatic duct cell, Nonstriated duct cell, Principal cell, Intercalated cell, Duct cell, Intestinal brush border cell, Exocrine gland striated duct cell, Gall bladder epithelial cell, Ductulus efferens nonciliated cell, Epididymal principal cell, and Epididymal basal cell; Extracellular matrix cells including, but not limited to: Ameloblast epithelial cell, Planum semilunatum epithelial cell of vestibular system of ear, Organ of Corti interdental epithelial cell, Loose connective tissue fibroblasts, Corneal fibroblasts, Tendon fibroblasts, Bone marrow reticular tissue fibroblasts, Other nonepithelial fibroblasts, Pericyte, Nucleus pulposus cell of intervertebral disc, Cementoblast/cementocyte, Odontoblast/odontocyte, Hyaline cartilage chondrocyte, Fibrocartilage chondrocyte, Elastic cartilage chondrocyte, Osteoblast/osteocyte, Osteoprogenitor cell, Hyalocyte of vitreous body of eye, Stellate cell of perilymphatic space of ear, Hepatic stellate cell, and Pancreatic stelle cell; Contractile cells including, but not limited to: Skeletal muscle cell, Satellite cell, Heart muscle cells, Smooth muscle cell, Myoepithelial cell of iris, and Myoepithelial cell of exocrine glands; Blood and immune system cells including, but not limited to: Erythrocyte, Megakaryocyte, Monocyte, Connective tissue macrophage, Epidermal Langerhans cell, Osteoclast, Dendritic cell, Microglial cell, Neutrophil granulocyte, Eosinophil granulocyte, Basophil granulocyte, Hybridoma cell, Mast cell, Helper T cell, Suppressor T cell, Cytotoxic T cell, Natural Killer T cell, B cell, Natural killer cell, Reticulocyte, Stem cells, and committed progenitors for the blood and immune system; Germ cells including, but not limited to: Oogonium/Oocyte, Spermatid, Spermatocyte, Spermatogonium cell, and Spermatozoon; Nurse cell including, but not limited to: Ovarian follicle cell, and Sertoli cell, Thymus epithelial cell; Interstitial cells including, but not limited to: Interstitial kidney cells and any combination of the foregoing.

Non-limiting examples of other known biologics include, but are not limited to: Abbosynagis, Abegrin, Actemra, AFP-Cide, Antova, Arzerra, Aurexis, Avastin, Benlysta, Bexxar, Blontress, Bosatria, Campath, CEA-Cide, CEA-Scan, Cimzia, Cyramza, Ektomab, Erbitux, FibriScint, Gazyva, Herceptin, hPAM4-Cide, HumaSPECT, HuMax-CD4, HuMax-EGFr, Humira, HuZAF, Hybri-ceaker, Ilaris, Indimacis-125, Kadcyla, Lemtrada, LeukArrest, LeukoScan, Lucentis, Lymphomun, LymphoScan, LymphoStat-B, MabThera, Mycograb, Mylotarg, Myoscint, NeutroSpec, Numax, Nuvion, Omnitarg, Opdivo, Orthoclone OKT3, OvaRex, Panorex, Prolia, Prostascint, Raptiva, Remicade, Removab, Rencarex, ReoPro, Rexomun, Rituxan, RoActemra, Scintimun, Simponi, Simulect, Soliris, Stelara, Synagis, Tactress, Theracim, Theragyn, Theraloc, Tysabri, Vectibix, Verluma, Xolair, Yervoy, Zenapax, and Zevalin and combinations thereof.

Non-limiting examples of known Monoclonal antibodies include, but are not limited to: 3F8, 8H9, Abagovomab, Abciximab, Abituzumab, Abrilumab, Actoxumab, Adalimumab, Adecatumumab, Aducanumab, Afasevikumab, Afelimomab, Afutuzumab, Alacizumab pegol, ALD518, ALD403, Alemtuzumab, Alirocumab, Altumomab pentetate, Amatuximab, AMG 334, Anatumomab mafenatox, Anetumab ravtansine, Anifrolumab, Anrukinzumab, Apolizumab, Arcitumomab, Ascrinvacumab, Aselizumab, Atezolizumab, Atinumab, Atlizumab, Atorolimumab, Avelumab, Bapineuzumab, Basiliximab, Bavituximab, Bectumomab, Begelomab, Belimumab, Benralizumab, Bertilimumab, Besilesomab, Bevacizumab, Bezlotoxumab, Biciromab, Bimagrumab, Bimekizumab, Bivatuzumab mertansine, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Canakinumab, Cantuzumab mertansine, Cantuzumab ravtansine, Caplacizumab, Capromab pendetide, Carlumab, Carotuximab, Catumaxomab, cBR96-doxorubicin immunoconjugate, Cedelizumab, Cergutuzumab amunaleukin, Certolizumab pegol, Cetuximab, Citatuzumab bogatox, Cixutumumab, Clazakizumab, Clenoliximab, Clivatuzumab tetraxetan, Codrituzumab, Coltuximab ravtansine, Conatumumab, Concizumab, CR6261, Crenezumab, Crotedumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dinutuximab, Diridavumab, Domagrozumab, Dorlimomab aritox, Drozitumab, Duligotumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Emicizumab, Enavatuzumab, Enfortumab vedotin, Enlimomab pegol, Enoblituzumab, Enokizumab, Enoticumab, Ensituximab, Epitumomab cituxetan, Epratuzumab, Erenumab, Erlizumab, Ertumaxomab, Etaracizumab, Etrolizumab, Evinacumab, Evolocumab, Exbivirumab, Fanolesomab, Faralimomab, Farletuzumab, Fasinumab, FBTA05, Felvizumab, Fezakinumab, Fibatuzumab, Ficlatuzumab, Figitumumab, Firivumab, Flanvotumab, Fletikumab, Fontolizumab, Foralumab, Foravirumab, Fresolimumab, Fulranumab, Futuximab, Galcanezumab, Galiximab, Ganitumab, Gantenerumab, Gavilimomab, Gemtuzumab ozogamicin, Gevokizumab, Girentuximab, Glembatumumab vedotin, Golimumab, Gomiliximab, Guselkumab, Ibalizumab, Ibritumomab tiuxetan, Icrucumab, Idarucizumab, Igovomab, IMA-638, IMAB362, Imalumab, Imciromab, Imgatuzumab, Inclacumab, Indatuximab ravtansine, Indusatumab vedotin, Inebilizumab, Infliximab, Inolimomab, Inotuzumab ozogamicin, Intetumumab, Ipilimumab, Iratumumab, Isatuximab, Itolizumab, Ixekizumab, Keliximab, Labetuzumab, Lambrolizumab, Lampalizumab, Lanadelumab, Landogrozumab, Laprituximab emtansine, LBR-101/PF0442g7429, Lebrikizumab, Lemalesomab, Lendalizumab, Lenzilumab, Lerdelimumab, Lexatumumab, Libivirumab, Lifastuzumab vedotin, Ligelizumab, Lilotomab satetraxetan, Lintuzumab, Lirilumab, Lodelcizumab, Lokivetmab, Lorvotuzumab mertansine, Lucatumumab, Lulizumab pegol, Lumiliximab, Lumretuzumab, LY2951742, Mapatumumab, Margetuximab, Maslimomab, Matuzumab, Mavrilimumab, Mepolizumab, Metelimumab, Milatuzumab, Minretumomab, Mirvetuximab soravtansine, Mitumomab, Mogamulizumab, Monalizumab, Morolimumab, Motavizumab, Moxetumomab pasudotox, Muromonab-CD3, Nacolomab tafenatox, Nam ilumab, Naptumomab estafenatox, Naratuximab emtansine, Narnatumab, Natalizumab, Navicixizumab, Navivumab, Nebacumab, Necitumumab, Nemolizumab, Nerelimomab, Nesvacumab, Nimotuzumab, Nivolumab, Nofetumomab merpentan, Obiltoxaximab, Obinutuzumab, Ocaratuzumab, Ocrelizumab, Odulimomab, Ofatumumab, Olaratumab, Olokizumab, Omalizumab, Onartuzumab, Ontuxizumab, Opicinumab, Oportuzumab monatox, Oregovomab, Orticumab, Otelixizumab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab vedotin, Pintumomab, Placulumab, Plozalizumab, Pogalizumab, Polatuzumab vedotin, Ponezumab, Prezalizumab, Priliximab, Pritoxaximab, Pritumumab, PRO 140, Quilizumab, Racotumomab, Radretumab, Rafivirumab, Ralpancizumab, Ramucirumab, Ranibizumab, Raxibacumab, Refanezumab, Regavirumab, Reslizumab, Rilotumumab, Rinucumab, Risankizumab, Rituximab, Rivabazumab pegol, Robatumumab, Roledumab, Romosozumab, Rontalizumab, Rovalpituzumab tesirine, Rovelizumab, Ruplizumab, Sacituzumab govitecan, Samalizumab, Sapelizumab, Sarilumab, Satumomab pendetide, Secukinumab, Seribantumab, Setoxaximab, Sevirumab, SGN-CD19A, SGN-CD33A, Sibrotuzumab, Sifalimumab, Siltuximab, Simtuzumab, Siplizumab, Sirukumab, Sofituzumab vedotin, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tamtuvetmab, Tanezumab, Taplitumomab paptox, Tarextumab, Tefibazumab, Telimomab aritox, Tenatumomab, Teneliximab, Teplizumab, Teprotumumab, Tesidolumab, Tetulomab, Tezepelumab, TGN1412, Ticilimumab, Tigatuzumab, Tildrakizumab, Timolumab, Tisotumab vedotin, TNX-650, Tocilizumab, Toralizumab, Tosatoxumab, Tositumomab, Tovetumab, Tralokinumab, Trastuzumab, Trastuzumab emtansine, TRBS07, Tregalizumab, Tremelimumab, Trevogrumab, Tucotuzumab celmoleukin, Tuvirumab, Ublituximab, Ulocuplumab, Urelumab, Urtoxazumab, Ustekinumab, Utomilumab, Vadastuximab talirine, Vandortuzumab vedotin, Vantictumab, Vanucizumab, Vapaliximab, Varlilumab, Vatelizumab, Vedolizumab, Veltuzumab, Vepalimomab, Vesencumab, Visilizumab, Vobarilizumab, Volociximab, Vorsetuzumab mafodotin, Votumumab, Xentuzumab, Zalutumumab, Zanolimumab, Zatuximab, Ziralimumab, and Zolimomab aritox and combinations thereof.

Examples of vaccines developed for viral diseases include, but are not limited to: Hepatitis A vaccine, Hepatitis B vaccine, Hepatitis E vaccine, HPV vaccine, Influenza vaccine, Japanese encephalitis vaccine, MMR vaccine, MMRV vaccine, Polio vaccine, Rabies vaccine, Rotavirus vaccine, Varicella vaccine, Shingles vaccine, Smallpox vaccine, Yellow Fever vaccine, Adenovirus vaccine, Coxsackie B virus vaccine, Cytomegalovirus vaccine, Dengue vaccine for humans, Eastern Equine encephalitis virus vaccine for humans, Ebola vaccine, Enterovirus 71 vaccine, Epstein-Barr vaccine, Hepatitis C vaccine, HIV vaccine, HTLV-1 T-lymphotropic leukemia vaccine for humans, Marburg virus disease vaccine, Norovirus vaccine, Respiratory syncytial virus vaccine for humans, Severe acute respiratory syndrome (SARS) vaccine, West Nile virus vaccine for humans; Examples of bacterial diseases include but are not limited to: Anthrax vaccines, DPT vaccine, Q fever vaccine, Hib vaccine, Tuberculosis (BCG) vaccine, Meningococcal vaccine, Typhoid vaccine, Pneumococcal conjugate vaccine, Pneumococcal polysaccharide vaccine, Cholera vaccine, Caries vaccine, Ehrlichiosis vaccine, Leprosy vaccine, Lyme disease vaccine, Staphylococcus aureus vaccine, Streptococcus pyogenes vaccine, Syphilis vaccine, Tularemia vaccine, and Yersinia pestis vaccine; Examples of parasitic diseases include, but are not limited to: Malaria vaccine, Schistosomiasis vaccine, Chagas disease vaccine, Hookworm vaccine, Onchocerciasis river blindness vaccine for humans, Trypanosomiasis vaccine, and Visceral leishmaniasis vaccine; Examples of non-infectious diseases include, but are not limited to: Alzheimer's disease amyloid protein vaccine, Breast cancer vaccine, Ovarian cancer vaccine, Prostate cancer vaccine, and Talimogene laherparepvec (T-VEC); also vaccines including, but not limited to the following trade names: ACAM2000, ActHIB, Adacel, Afluria, AFLURIA QUADRIVALENT, Agriflu, BCG Vaccine, BEXSERO, Biothrax, Boostrix, Cervarix, Comvax, DAPTACEL, DECAVAC, Engerix-B, FLUAD, Fluarix, Fluarix Quadrivalent, Flublok, Flucelvax, Flucelvax Quadrivalent, FluLaval, FluMist, FluMist Quadrivalent, Fluvirin, Fluzone Quadrivalent, Fluzone, Fluzone High-Dose and Fluzone Intradermal, Gardasil, Gardasil 9, Havrix, Hiberix, Imovax, Infanrix, IPOL, Ixiaro, JE-Vax, KINRIX, Menactra, MenHibrix, Menomune-A/C/Y/W-135, Menveo, M-M-R II, M-M-Vax, Pediarix, PedvaxHIB, Pentacel, Pneumovax 23, Poliovax, Prevnar, Prevnar 13, ProQuad, Quadracel, Quadrivalent, RabAvert, Recombivax HB, ROTARIX, RotaTeq, TENIVAC, TICE BCG, Tripedia, TRUMENBA, Twinrix, TYPHIM Vi, VAQTA, Varivax, Vaxchora, Vivotif, YF-Vax, Zostavax, and combinations thereof.

Examples of injectable drugs include, but are not limited to: Ablavar (Gadofosveset Trisodium Injection), Abarelix Depot, Abobotulinumtoxin A Injection (Dysport), ABT-263, ABT-869, ABX-EFG, Accretropin (Somatropin Injection), Acetadote (Acetylcysteine Injection), Acetazolamide Injection (Acetazolamide Injection), Acetylcysteine Injection (Acetadote), Actemra (Tocilizumab Injection), Acthrel (Corticorelin Ovine Triflutate for Injection), Actummune, Activase, Acyclovir for Injection (Zovirax Injection), Adacel, Adalimumab, Adenoscan (Adenosine Injection), Adenosine Injection (Adenoscan), Adrenaclick, AdreView (Iobenguane 1123 Injection for Intravenous Use), Afluria, Ak-Fluor (Fluorescein Injection), Aldurazyme (Laronidase), Alglucerase Injection (Ceredase), Alkeran Injection (Melphalan Hcl Injection), Allopurinol Sodium for Injection (Aloprim), Aloprim (Allopurinol Sodium for Injection), Alprostadil, Alsuma (Sumatriptan Injection), ALTU-238, Amino Acid Injections, Aminosyn, Apidra, Apremilast, Alprostadil Dual Chamber System for Injection (Caverject Impulse), AMG 009, AMG 076, AMG 102, AMG 108, AMG 114, AMG 162, AMG 220, AMG 221, AMG 222, AMG 223, AMG 317, AMG 379, AMG 386, AMG 403, AMG 477, AMG 479, AMG 517, AMG 531, AMG 557, AMG 623, AMG 655, AMG 706, AMG 714, AMG 745, AMG 785, AMG 811, AMG 827, AMG 837, AMG 853, AMG 951, Amiodarone HCI Injection (Amiodarone HCI Injection), Amobarbital Sodium Injection (Amytal Sodium), Amytal Sodium (Amobarbital Sodium Injection), Anakinra, Anti-Abeta, Anti-Beta7, Anti-Beta20, Anti-CD4, Anti-CD20, Anti-CD40, Anti-IFNalpha, Anti-IL13, Anti-OX40L, Anti-oxLDS, Anti-NGF, Anti-NRP1, Arixtra, Amphadase (Hyaluronidase Inj), Ammonul (Sodium Phenylacetate and Sodium Benzoate Injection), Anaprox, Anzemet Injection (Dolasetron Mesylate Injection), Apidra (Insulin Glulisine [rDNA origin] Inj), Apomab, Aranesp (darbepoetin alfa), Argatroban (Argatroban Injection), Arginine Hydrochloride Injection (R-Gene 10, Aristocort, Aristospan, Arsenic Trioxide Injection (Trisenox), Articane HCI and Epinephrine Injection (Septocaine), Arzerra (Ofatumumab Injection), Asclera (Polidocanol Injection), Ataluren, Ataluren-DMD, Atenolol Inj (Tenormin I.V. Injection), Atracurium Besylate Injection (Atracurium Besylate Injection), Avastin, Azactam Injection (Aztreonam Injection), Azithromycin (Zithromax Injection), Aztreonam Injection (Azactam Injection), Baclofen Injection (Lioresal Intrathecal), Bacteriostatic Water (Bacteriostatic Water for Injection), Baclofen Injection (Lioresal Intrathecal), Bal in Oil Ampules (Dimercarprol Injection), BayHepB, BayTet, Benadryl, Bendamustine Hydrochloride Injection (Treanda), Benztropine Mesylate Injection (Cogentin), Betamethasone Injectable Suspension (Celestone Soluspan), Bexxar, Bicillin C-R 900/300 (Penicillin G Benzathine and Penicillin G Procaine Injection), Blenoxane (Bleomycin Sulfate Injection), Bleomycin Sulfate Injection (Blenoxane), Boniva Injection (Ibandronate Sodium Injection), Botox Cosmetic (OnabotulinumtoxinA for Injection), BR3-FC, Bravelle (Urofollitropin Injection), Bretylium (Bretylium Tosylate Injection), Brevital Sodium (Methohexital Sodium for Injection), Brethine, Briobacept, BTT-1023, Bupivacaine HCI, Byetta, Ca-DTPA (Pentetate Calcium Trisodium Inj), Cabazitaxel Injection (Jevtana), Caffeine Alkaloid (Caffeine and Sodium Benzoate Injection), Calcijex Injection (Calcitrol), Calcitrol (Calcijex Injection), Calcium Chloride (Calcium Chloride Injection 10%), Calcium Disodium Versenate (Edetate Calcium Disodium Injection), Campath (Altemtuzumab), Camptosar Injection (Irinotecan Hydrochloride), Canakinumab Injection (Ilaris), Capastat Sulfate (Capreomycin for Injection), Capreomycin for Injection (Capastat Sulfate), Cardiolite (Prep kit for Technetium Tc99 Sestamibi for Injection), Carticel, Cathflo, Cefazolin and Dextrose for Injection (Cefazolin Injection), Cefepime Hydrochloride, Cefotaxime, Ceftriaxone, Cerezyme, Carnitor Injection, Caverject, Celestone Soluspan, Celsior, Cerebyx (Fosphenytoin Sodium Injection), Ceredase (Alglucerase Injection), Ceretec (Technetium Tc99m Exametazime Injection), Certolizumab, CF-101, Chloramphenicol Sodium Succinate (Chloramphenicol Sodium Succinate Injection), Chloramphenicol Sodium Succinate Injection (Chloramphenicol Sodium Succinate), Cholestagel (Colesevelam HCL), Choriogonadotropin Alfa Injection (Ovidrel), Cimzia, Cisplatin (Cisplatin Injection), Clolar (Clofarabine Injection), Clomiphine Citrate, Clonidine Injection (Duraclon), Cogentin (Benztropine Mesylate Injection), Colistimethate Injection (Coly-Mycin M), Coly-Mycin M (Colistimethate Injection), Compath, Conivaptan Hcl Injection (Vaprisol), Conjugated Estrogens for Injection (Premarin Injection), Copaxone, Corticorelin Ovine Triflutate for Injection (Acthrel), Corvert (Ibutilide Fumarate Injection), Cubicin (Daptomycin Injection), CF-1 01, Cyanokit (Hydroxocobalamin for Injection), Cytarabine Liposome Injection (DepoCyt), Cyanocobalamin, Cytovene (ganciclovir), D.H.E. 45, Dacetuzumab, Dacogen (Decitabine Injection), Dalteparin, Dantrium IV (Dantrolene Sodium for Injection), Dantrolene Sodium for Injection (Dantrium IV), Daptomycin Injection (Cubicin), Darbepoietin Alfa, DDAVP Injection (Desmopressin Acetate Injection), Decavax, Decitabine Injection (Dacogen), Dehydrated Alcohol (Dehydrated Alcohol Injection), Denosumab Injection (Prolia), Delatestryl, Delestrogen, Delteparin Sodium, Depacon (Valproate Sodium Injection), Depo Medrol (Methylprednisolone Acetate Injectable Suspension), DepoCyt (Cytarabine Liposome Injection), DepoDur (Morphine Sulfate XR Liposome Injection), Desmopressin Acetate Injection (DDAVP Injection), Depo-Estradiol, Depo-Provera 104 mg/ml, Depo-Provera 150 mg/ml, Depo-Testosterone, Dexrazoxane for Injection, Intravenous Infusion Only (Totect), Dextrose/Electrolytes, Dextrose and Sodium Chloride Inj (Dextrose 5% in 0.9% Sodium Chloride), Dextrose, Diazepam Injection (Diazepam Injection), Digoxin Injection (Lanoxin Injection), Dilaudid-HP (Hydromorphone Hydrochloride Injection), Dimercarprol Injection (Bal in Oil Ampules), Diphenhydramine Injection (Benadryl Injection), Dipyridamole Injection (Dipyridamole Injection), DMOAD, Docetaxel for Injection (Taxotere), Dolasetron Mesylate Injection (Anzemet Injection), Doribax (Doripenem for Injection), Doripenem for Injection (Doribax), Doxercalciferol Injection (Hectorol Injection), Doxil (Doxorubicin Hcl Liposome Injection), Doxorubicin Hcl Liposome Injection (Doxil), Duraclon (Clonidine Injection), Duramorph (Morphine Injection), Dysport (Abobotulinumtoxin A Injection), Ecallantide Injection (Kalbitor), EC-Naprosyn (naproxen), Edetate Calcium Disodium Injection (Calcium Disodium Versenate), Edex (Alprostadil for Injection), Engerix, Edrophonium Injection (Enlon), Eliglustat Tartate, Eloxatin (Oxaliplatin Injection), Emend Injection (Fosaprepitant Dimeglumine Injection), Enalaprilat Injection (Enalaprilat Injection), Enlon (Edrophonium Injection), Enoxaparin Sodium Injection (Lovenox), Eovist (Gadoxetate Disodium Injection), Enbrel (etanercept), Enoxaparin, Epicel, Epinepherine, Epipen, Epipen Jr., Epratuzumab, Erbitux, Ertapenem Injection (Invanz), Erythropoieten, Essential Amino Acid Injection (Nephramine), Estradiol Cypionate, Estradiol Valerate, Etanercept, Exenatide Injection (Byetta), Evlotra, Fabrazyme (Adalsidase beta), Famotidine Injection, FDG (Fludeoxyglucose F 18 Injection), Feraheme (Ferumoxytol Injection), Feridex I.V. (Ferumoxides Injectable Solution), Fertinex, Ferumoxides Injectable Solution (Feridex I.V.), Ferumoxytol Injection (Feraheme), Flagyl Injection (Metronidazole Injection), Fluarix, Fludara (Fludarabine Phosphate), Fludeoxyglucose F 18 Injection (FDG), Fluorescein Injection (Ak-Fluor), Follistim AQ Cartridge (Follitropin Beta Injection), Follitropin Alfa Injection (Gonal-f RFF), Follitropin Beta Injection (Follistim AQ Cartridge), Folotyn (Pralatrexate Solution for Intravenous Injection), Fondaparinux, Forteo (Teriparatide (rDNA origin) Injection), Fostamatinib, Fosaprepitant Dimeglumine Injection (Emend Injection), Foscarnet Sodium Injection (Foscavir), Foscavir (Foscarnet Sodium Injection), Fosphenytoin Sodium Injection (Cerebyx), Fospropofol Disodium Injection (Lusedra), Fragmin, Fuzeon (enfuvirtide), GA101, Gadobenate Dimeglumine Injection (Multihance), Gadofosveset Trisodium Injection (Ablavar), Gadoteridol Injection Solution (ProHance), Gadoversetamide Injection (OptiMARK), Gadoxetate Disodium Injection (Eovist), Ganirelix (Ganirelix Acetate Injection), Gardasil, GC1008, GDFD, Gemtuzumab Ozogamicin for Injection (Mylotarg), Genotropin, Gentamicin Injection, GENZ-112638, Golimumab Injection (Simponi Injection), Gonal-f RFF (Follitropin Alfa Injection), Granisetron Hydrochloride (Kytril Injection), Gentamicin Sulfate, Glatiramer Acetate, Glucagen, Glucagon, HAE1, Haldol (Haloperidol Injection), Havrix, Hectorol Injection (Doxercalciferol Injection), Hedgehog Pathway Inhibitor, Heparin, Herceptin, hG-CSF, Humalog, Human Growth Hormone, Humatrope, HuMax, Humegon, Humira, Humulin, Ibandronate Sodium Injection (Boniva Injection), Ibuprofen Lysine Injection (NeoProfen), Ibutilide Fumarate Injection (Corvert), Idamycin PFS (Idarubicin Hydrochloride Injection), Idarubicin Hydrochloride Injection (Idamycin PFS), Ilaris (Canakinumab Injection), Imipenem and Cilastatin for Injection (Primaxin I.V.), Imitrex, Incobotulinumtoxin A for Injection (Xeomin), Increlex (Mecasermin [rDNA origin] Injection), Indocin IV (Indomethacin Inj), Indomethacin Inj (Indocin IV), Infanrix, Innohep, Insulin, Insulin Aspart [rDNA origin] Inj (NovoLog), Insulin Glargine [rDNA origin] Injection (Lantus), Insulin Glulisine [rDNA origin] Inj (Apidra), Interferon alfa-2b, Recombinant for Injection (Intron A), Intron A (Interferon alfa-2b, Recombinant for Injection), Invanz (Ertapenem Injection), Invega Sustenna (Paliperidone Palmitate Extended-Release Injectable Suspension), Invirase (saquinavir mesylate), lobenguane 1123 Injection for Intravenous Use (AdreView), lopromide Injection (Ultravist), loversol Injection (Optiray Injection), Iplex (Mecasermin Rinfabate [rDNA origin] Injection), Iprivask, Irinotecan Hydrochloride (Camptosar Injection), Iron Sucrose Injection (Venofer), Istodax (Romidepsin for Injection), Itraconazole Injection (Sporanox Injection), Jevtana (Cabazitaxel Injection), Jonexa, Kalbitor (Ecallantide Injection), KCL in D5NS (Potassium Chloride in 5% Dextrose and Sodium Chloride Injection), KCL in D5W, KCL in NS, Kenalog 10 Injection (Triamcinolone Acetonide Injectable Suspension), Kepivance (Palifermin), Keppra Injection (Levetiracetam), Keratinocyte, KFG, Kinase Inhibitor, Kineret (Anakinra), Kinlytic (Urokinase Injection), Kinrix, Klonopin (clonazepam), Kytril Injection (Granisetron Hydrochloride), lacosamide Tablet and Injection (Vimpat), Lactated Ringer's, Lanoxin Injection (Digoxin Injection), Lansoprazole for Injection (Prevacid I.V.), Lantus, Leucovorin Calcium (Leucovorin Calcium Injection), Lente (L), Leptin, Levemir, Leukine Sargramostim, Leuprolide Acetate, Levothyroxine, Levetiracetam (Keppra Injection), Lovenox, Levocarnitine Injection (Carnitor Injection), Lexiscan (Regadenoson Injection), Lioresal Intrathecal (Baclofen Injection), Liraglutide [rDNA] Injection (Victoza), Lovenox (Enoxaparin Sodium Injection), Lucentis (Ranibizumab Injection), Lumizyme, Lupron (Leuprolide Acetate Injection), Lusedra (Fospropofol Disodium Injection), Maci, Magnesium Sulfate (Magnesium Sulfate Injection), Mannitol Injection (Mannitol IV), Marcaine (Bupivacaine Hydrochloride and Epinephrine Injection), Maxipime (Cefepime Hydrochloride for Injection), MDP Multidose Kit of Technetium Injection (Technetium Tc99m Medronate Injection), Mecasermin [rDNA origin] Injection (Increlex), Mecasermin Rinfabate [rDNA origin] Injection (Iplex), Melphalan Hcl Injection (Alkeran Injection), Methotrexate, Menactra, Menopur (Menotropins Injection), Menotropins for Injection (Repronex), Methohexital Sodium for Injection (Brevital Sodium), Methyldopate Hydrochloride Injection, Solution (Methyldopate Hcl), Methylene Blue (Methylene Blue Injection), Methylprednisolone Acetate Injectable Suspension (Depo Medrol), MetMab, Metoclopramide Injection (Reglan Injection), Metrodin (Urofollitropin for Injection), Metronidazole Injection (Flagyl Injection), Miacalcin, Midazolam (Midazolam Injection), Mimpara (Cinacalet), Minocin Injection (Minocycline Inj), Minocycline Inj (Minocin Injection), Mipomersen, Mitoxantrone for Injection Concentrate (Novantrone), Morphine Injection (Duramorph), Morphine Sulfate XR Liposome Injection (DepoDur), Morrhuate Sodium (Morrhuate Sodium Injection), Motesanib, Mozobil (Plerixafor Injection), Multihance (Gadobenate Dimeglumine Injection), Multiple Electrolytes and Dextrose Injection, Multiple Electrolytes Injection, Mylotarg (Gemtuzumab Ozogamicin for Injection), Myozyme (Alglucosidase alfa), Nafcillin Injection (Nafcillin Sodium), Nafcillin Sodium (Nafcillin Injection), Naltrexone XR Inj (Vivitrol), Naprosyn (naproxen), NeoProfen (Ibuprofen Lysine Injection), Nandrol Decanoate, Neostigmine Methylsulfate (Neostigmine Methylsulfate Injection), NEO-GAA, NeoTect (Technetium Tc 99m Depreotide Injection), Nephramine (Essential Amino Acid Injection), Neulasta (pegfilgrastim), Neupogen (Filgrastim), Novolin, Novolog, NeoRecormon, Neutrexin (Trimetrexate Glucuronate Inj), NPH (N), Nexterone (Amiodarone HCl Injection), Norditropin (Somatropin Injection), Normal Saline (Sodium Chloride Injection), Novantrone (Mitoxantrone for Injection Concentrate), Novolin 70/30 Innolet (70% NPH, Human Insulin Isophane Suspension and 30% Regular, Human Insulin Injection), NovoLog (Insulin Aspart [rDNA origin] Inj), Nplate (romiplostim), Nutropin (Somatropin (rDNA origin) for Inj), Nutropin AQ, Nutropin Depot (Somatropin (rDNA origin) for Inj), Octreotide Acetate Injection (Sandostatin LAR), Ocrelizumab, Ofatumumab Injection (Arzerra), Olanzapine Extended Release Injectable Suspension (Zyprexa Relprevv), Omnitarg, Omnitrope (Somatropin [rDNA origin] Injection), Ondansetron Hydrochloride Injection (Zofran Injection), OptiMARK (Gadoversetamide Injection), Optiray Injection (loversol Injection), Orencia, Osmitrol Injection in Aviva (Mannitol Injection in Aviva Plastic Vessel 250), Osmitrol Injection in Viaflex (Mannitol Injection in Viaflex Plastic Vessel 250), Osteoprotegrin, Ovidrel (Choriogonadotropin Alfa Injection), Oxacillin (Oxacillin for Injection), Oxaliplatin Injection (Eloxatin), Oxytocin Injection (Pitocin), Paliperidone Palmitate Extended-Release Injectable Suspension (Invega Sustenna), Pamidronate Disodium Injection (Pam idronate Disodium Injection), Panitumumab Injection for Intravenous Use (Vectibix), Papaverine Hydrochloride Injection (Papaverine Injection), Papaverine Injection (Papaverine Hydrochloride Injection), Parathyroid Hormone, Paricalcitol Injection Fliptop Vial (Zemplar Injection), PARP Inhibitor, Pediarix, PEGIntron, Peginterferon, Pegfilgrastim, Penicillin G Benzathine and Penicillin G Procaine, Pentetate Calcium Trisodium Inj (Ca-DTPA), Pentetate Zinc Trisodium Injection (Zn-DTPA), Pepcid Injection (Famotidine Injection), Pergonal, Pertuzumab, Phentolamine Mesylate (Phentolamine Mesylate for Injection), Physostigmine Salicylate (Physostigmine Salicylate (injection)), Physostigmine Salicylate (injection) (Physostigmine Salicylate), Piperacillin and Tazobactam Injection (Zosyn), Pitocin (Oxytocin Injection), Plasma-Lyte 148 (Multiple Electrolytes Inj), Plasma-Lyte 56 and Dextrose (Multiple Electrolytes and Dextrose Injection in Viaflex, Plastic Vessel 250), PlasmaLyte, Plerixafor Injection (Mozobil), Polidocanol Injection (Asclera), Potassium Chloride, Pralatrexate Solution for Intravenous Injection (Folotyn), Pramlintide Acetate Injection (Symlin), Premarin Injection (Conjugated Estrogens for Injection), Prep kit for Technetium Tc99 Sestamibi for Injection (Cardiolite), Prevacid I.V. (Lansoprazole for Injection), Primaxin I.V. (Imipenem and Cilastatin for Injection), Prochymal, Procrit, Progesterone, ProHance (Gadoteridol Injection Solution), Prolia (Denosumab Injection), Promethazine HCI Injection (Promethazine Hydrochloride Injection), Propranolol Hydrochloride Injection (Propranolol Hydrochloride Injection), Quinidine Gluconate Injection (Quinidine Injection), Quinidine Injection (Quinidine Gluconate Injection), R-Gene 10 (Arginine Hydrochloride Injection), Ranibizumab Injection (Lucentis), Ranitidine Hydrochloride Injection (Zantac Injection), Raptiva, Reclast (Zoledronic Acid Injection), Recombivarix HB, Regadenoson Injection (Lexiscan), Reglan Injection (Metoclopramide Injection), Remicade, Renagel, Renvela (Sevelamer Carbonate), Repronex (Menotropins for Injection), Retrovir IV (Zidovudine Injection), rhApo2L/TRAIL, Ringer's and 5% Dextrose Injection (Ringers in Dextrose), Ringer's Injection (Ringers Injection), Rituxan, Rituximab, Rocephin (ceftriaxone), Rocuronium Bromide Injection (Zemuron), Roferon-A (interferon alfa-2a), Romazicon (flumazenil), Romidepsin for Injection (Istodax), Saizen (Somatropin Injection), Sandostatin LAR (Octreotide Acetate Injection), Sclerostin Ab, Sensipar (cinacalcet), Sensorcaine (Bupivacaine HCI Injections), Septocaine (Articane HCI and Epinephrine Injection), Serostim LQ (Somatropin (rDNA origin) Injection), Simponi Injection (Golimumab Injection), Sodium Acetate (Sodium Acetate Injection), Sodium Bicarbonate (Sodium Bicarbonate 5% Injection), Sodium Lactate (Sodium Lactate Injection in AVIVA), Sodium Phenylacetate and Sodium Benzoate Injection (Ammonul), Somatropin (rDNA origin) for Inj (Nutropin), Sporanox Injection (Itraconazole Injection), Stelara Injection (Ustekinumab), Stemgen, Sufenta (Sufentanil Citrate Injection), Sufentanil Citrate Injection (Sufenta), Sumavel, Sumatriptan Injection (Alsuma), Symlin, Symlin Pen, Systemic Hedgehog Antagonist, Synvisc-One (Hylan G-F 20 Single Intra-articular Injection), Tarceva, Taxotere (Docetaxel for Injection), Technetium Tc 99m, Telavancin for Injection (Vibativ), Temsirolimus Injection (Torisel), Tenormin I.V. Injection (Atenolol Inj), Teriparatide (rDNA origin) Injection (Forteo), Testosterone Cypionate, Testosterone Enanthate, Testosterone Propionate, Tev-Tropin (Somatropin, rDNA Origin, for Injection), tgAAC94, Thallous Chloride, Theophylline, Thiotepa (Thiotepa Injection), Thymoglobulin (Anti-Thymocyte Globulin (Rabbit), Thyrogen (Thyrotropin Alfa for Injection), Ticarcillin Disodium and Clavulanate Potassium Galaxy (Timentin Injection), Tigan Injection (Trimethobenzamide Hydrochloride Injectable), Timentin Injection (Ticarcillin Disodium and Clavulanate Potassium Galaxy), TNKase, Tobramycin Injection (Tobramycin Injection), Tocilizumab Injection (Actemra), Torisel (Temsirolimus Injection), Totect (Dexrazoxane for Injection, Intravenous Infusion Only), Trastuzumab-DM1, Travasol (Amino Acids (Injection)), Treanda (Bendamustine Hydrochloride Injection), Trelstar (Triptorelin Pamoate for Injectable Suspension), Triamcinolone Acetonide, Triamcinolone Diacetate, Triamcinolone Hexacetonide Injectable Suspension (Aristospan Injection 20 mg), Triesence (Triamcinolone Acetonide Injectable Suspension), Trimethobenzamide Hydrochloride Injectable (Tigan Injection), Trimetrexate Glucuronate Inj (Neutrexin), Triptorelin Pamoate for Injectable Suspension (Trelstar), Twinject, Trivaris (Triamcinolone Acetonide Injectable Suspension), Trisenox (Arsenic Trioxide Injection), Twinrix, Typhoid Vi, Ultravist (Iopromide Injection), Urofollitropin for Injection (Metrodin), Urokinase Injection (Kinlytic), Ustekinumab (Stelara Injection), Ultralente (U), Valium (diazepam), Valproate Sodium Injection (Depacon), Valtropin (Somatropin Injection), Vancomycin Hydrochloride (Vancomycin Hydrochloride Injection), Vancomycin Hydrochloride Injection (Vancomycin Hydrochloride), Vaprisol (Conivaptan Hcl Injection), VAQTA, Vasovist (Gadofosveset Trisodium Injection for Intravenous Use), Vectibix (Panitumumab Injection for Intravenous Use), Venofer (Iron Sucrose Injection), Verteporfin Inj (Visudyne), Vibativ (Telavancin for Injection), Victoza (Liraglutide [rDNA] Injection), Vimpat (lacosamide Tablet and Injection), Vinblastine Sulfate (Vinblastine Sulfate Injection), Vincasar PFS (Vincristine Sulfate Injection), Victoza, Vincristine Sulfate (Vincristine Sulfate Injection), Visudyne (Verteporfin Inj), Vitamin B-12, Vivitrol (Naltrexone XR Inj), Voluven (Hydroxyethyl Starch in Sodium Chloride Injection), Xeloda, Xenical (orlistat), Xeomin (Incobotulinumtoxin A for Injection), Xolair, Zantac Injection (Ranitidine Hydrochloride Injection), Zemplar Injection (Paricalcitol Injection Fliptop Vial), Zemuron (Rocuronium Bromide Injection), Zenapax (daclizumab), Zevalin, Zidovudine Injection (Retrovir IV), Zithromax Injection (Azithromycin), Zn-DTPA (Pentetate Zinc Trisodium Injection), Zofran Injection (Ondansetron Hydrochloride Injection), Zingo, Zoledronic Acid for Inj (Zometa), Zoledronic Acid Injection (Reclast), Zometa (Zoledronic Acid for Inj), Zosyn (Piperacillin and Tazobactam Injection), Zyprexa Relprevv (Olanzapine Extended Release Injectable Suspension) and combinations thereof.

### Notice

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims.

## Claims

1. A stopper (40) for use in an injector device (10), the stopper having an outer side configured for engagement with an interior bore of an injector device barrel (20), the stopper (40) comprising:
an elastomer body (240); and
a barrier (242) coupled to the elastomer body (240), the barrier (242) having an inner surface oriented toward the elastomer body (240) and an outer surface oriented away from the elastomer body (240), the barrier (242) including a first layer (402) of a first material and a second layer (404) of a second material, the first layer (402) being configured to be activatable by an energy source and the second layer (404) configured to be less activatable by the energy source than the first layer, and wherein:
the barrier (242) has at least one micro feature (400) formed in the barrier (242) by activating the first layer (402) with the energy source to cause a change in volume of the first layer upon being activated by the energy source, the at least one micro feature (400) including one or both of: a micro groove (400A) and/or a micro rib (400P).

2. The stopper (40) of claim 1, wherein the energy source includes at least one of a laser energy source, an RF energy source, a vibrational energy source, and a thermal energy source.

3. The stopper (40) of any preceding claim, wherein at least one of:
the first layer (402) is positioned under the second layer (404);
the first layer (402) is microporous and defines a first porosity and the second layer (404) has a lower porosity than the first layer;
the first material and/or the second material includes a fluoropolymer, optionally polytetrafluoroethylene (PTFE) or expanded PTFE (ePTFE); and
at least one of the first material of the first layer (402) and the second material of the second layer (404) includes a thermoplastic material.

4. The stopper of any preceding claim, wherein the barrier (242) has a thickness between 1 µm and 200 µm.

5. The stopper (40) of any preceding claim, wherein at least one of:
the second layer (404) is **characterized by** a higher melt temperature than the first layer; and
the second layer (404) is **characterized by** a higher dimensional stability than the first layer (402).

6. The stopper (40) of any preceding claim wherein the micro groove (400A) has a depth from 0.25 µm to 50 µm, and optionally from 0.25 µm to 0.5 µm and a width from 0.25 µm to 50 µm, and optionally from 0.25 µm to 0.5 µm; and/or
wherein the micro rib (400P) has a height from 0.25 µm to 50 µm, and optionally from 0.25 µm to 0.5 µm and a width from 0.25 µm to 50 µm, and optionally from 0.25 µm to 0.5 µm.

7. The stopper of any preceding claim, wherein at least one of:
the micro groove (400A) and/or the micro rib (400P) extends in a circumferential direction;
the micro groove (400A) and/or the micro rib (400P) extends in a helical direction.

8. The stopper of any preceding claim, wherein the micro groove (400A) has a base and two sides, and further wherein one of the two sides defines the micro rib (400P); optionally
wherein material forming the micro rib (400P) has a higher density than material forming the base of the micro groove (400A), or
wherein material forming the micro rib (400P) has a lower density than material forming the base of the micro groove (400A).

9. The stopper of any one of claims 1 to 8, wherein the first layer (402) includes a material configured to increase in volume upon being activated by the energy source, and further wherein the micro rib (400P) corresponds to a portion of the first layer (402) that has been increased in volume by being activated by the energy source, or
wherein the first layer (402) includes a material configured to be removed upon being activated by the energy source, and further wherein the micro groove (400A) corresponds to a portion of the first layer (402) that has been removed by being activated by the energy source.

10. The stopper of any preceding claim, wherein the barrier (242) has been bonded to the elastomer body (240) by activating the first layer (402) with the energy source.

11. The stopper of any preceding claim, wherein at least one of:
the micro groove (400A) and/or micro rib (400P) has been formed following coupling of the barrier (242) to the elastomer body (240);
the micro feature (400) is formed on the outer surface of the barrier (242);
the micro feature (400) is formed on the inner surface of the barrier (242);
the porosity of a portion of the first layer (402) corresponding to the micro groove (400A) and/or micro rib (400P) has been modified following coupling of the barrier (242) to the elastomer body (240).

12. The stopper of any preceding claim, wherein the first material of the first layer (402) includes a filler configured to increase absorption of light energy and/or radiofrequency energy of the first material, optionally
wherein the filler includes at least one of fluorinated ethylene propylene (FEP) and ethylene tetrafluoroethylene (ETFE).

13. A method of making a stopper (40) for use in an injector device (10), the stopper (40) having a seal surface configured for engagement with an interior bore of an injector device barrel (20), the method comprising:
activating a first layer (402) of a barrier (242) of the stopper (40) with an energy source to cause a change in volume of the first layer and form at least one micro feature (400), the barrier (242) having an inner surface and an outer surface, the barrier (242) including a first layer (402) of a first material and a second layer (404) of a second material, the micro feature (400) including one or both of: a micro groove (400A) and/or
a micro rib (400P); and
coupling the barrier (242) to an elastomer body (240).

14. The method of claim 13, wherein at least one of:
the second layer (404) is positioned over the first layer (402), and further wherein the first layer (402) is activated through the second layer (404);
the first layer (402) includes FEP and the second layer (404) includes PTFE;
the barrier (242) is coupled to the elastomer body (240) during formation of the at least one micro feature (400).

15. The method of claims 13 or 14, wherein the energy source includes at least one of a laser energy source, an RF energy source, a vibrational energy source, and a thermal energy source.

16. The method of any of claims 13 to 15, wherein at least one of:
forming the at least one micro feature (400) includes cooling the barrier (242) after activating the first layer (402);
simultaneously forming the micro groove (400A) and micro rib (400P), optionally by causing melted portions of the barrier (242) to reflow and resolidify;
activating a first layer (402) of the barrier (242) of the stopper (40) with the energy source to form at least one micro feature (400) includes inducing relative movement between the energy source and the stopper (40), the movement optionally including one or both of linear movement or rotational movement.

17. The method of any of claims 13 to 16, wherein at least one of:
the at least one micro feature (400) is formed prior to coupling the barrier (242) to the elastomer body (240);
the at least one micro feature (400) is formed with the barrier (242) in sheet form;
the at least one micro feature (400) is formed after coupling the barrier (242) to the elastomer body (240);
the micro feature (400) is formed on the outer surface of the barrier (242); and
the micro feature (400) is formed on the inner surface of the barrier (242).

## Patentansprüche

1. Anschlag (40) zur Verwendung in einer Injektorvorrichtung (10), wobei der Anschlag eine Außenseite aufweist, die zum Eingriff mit einer Innenbohrung eines Injektorvorrichtungszylinders (20) konfiguriert ist, wobei der Anschlag (40) umfasst:
einen Elastomerkörper (240); und
eine Barriere (242), die an den Elastomerkörper (240) gekoppelt ist, wobei die Barriere (242) eine Innenfläche, die zum Elastomerkörper (240) gerichtet ist, und eine Außenfläche, die vom Elastomerkörper (240) weg gerichtet ist, aufweist, wobei die Barriere (242) eine erste Schicht (402) aus einem ersten Material und eine zweite Schicht (404) aus einem zweiten Material beinhaltet, wobei die erste Schicht (402) so konfiguriert ist, dass sie durch eine Energiequelle aktiviert wird, und die zweite Schicht (404) so konfiguriert ist, dass sie durch die Energiequelle weniger aktivierbar ist als die erste Schicht, und wobei:
die Barriere (242) mindestens ein Mikromerkmal (400) aufweist, das in der Barriere (242) ausgebildet ist, indem die erste Schicht (402) mit der Energiequelle aktiviert wird, um eine Änderung des Volumens der ersten Schicht zu bewirken, wenn sie durch die Energiequelle aktiviert wird, wobei das mindestens eine Mikromerkmal (400) eines oder beide von: einer Mikronut (400A) und/oder einer Mikrorippe (400P) beinhaltet.

2. Anschlag (40) nach Anspruch 1, wobei die Energiequelle mindestens eine von einer Laserenergiequelle, einer HF-Energiequelle, einer Schwingungsenergiequelle und einer thermischen Energiequelle beinhaltet.

3. Anschlag (40) nach einem der vorhergehenden Ansprüche, wobei mindestens eines der Folgenden zutrifft:
die erste Schicht (402) ist unter der zweiten Schicht (404) positioniert;
die erste Schicht (402) ist mikroporös und definiert eine erste Porosität, und die zweite Schicht (404) weist eine geringere Porosität als die erste Schicht auf;
das erste Material und/oder das zweite Material beinhalten ein Fluorpolymer, optional Polytetrafluorethylen (PTFE) oder expandiertes PTFE (ePTFE); und
mindestens eines von dem ersten Material der ersten Schicht (402) und dem zweiten Material der zweiten Schicht (404) beinhaltet ein thermoplastisches Material.

4. Anschlag nach einem der vorhergehenden Ansprüche, wobei die Barriere (242) eine Dicke zwischen 1 µm und 200 µm aufweist.

5. Anschlag (40) nach einem der vorhergehenden Ansprüche, wobei mindestens eines der Folgenden zutrifft:
die zweite Schicht (404) ist durch eine höhere Schmelztemperatur gekennzeichnet als die erste Schicht; und
die zweite Schicht (404) ist durch eine höhere Dimensionsstabilität gekennzeichnet als die erste Schicht (402).

6. Anschlag (40) nach einem der vorhergehenden Ansprüche, wobei die Mikronut (400A) eine Tiefe von 0,25 µm bis 50 µm und optional von 0,25 µm bis 0,5 µm und eine Breite von 0,25 µm bis 50 µm und optional von 0,25 µm bis 0,5 µm aufweist; und/oder
wobei die Mikrorippe (400P) eine Höhe von 0,25 µm bis 50 µm und optional von 0,25 µm bis 0,5 µm und eine Breite von 0,25 µm bis 50 µm und optional von 0,25 µm bis 0,5 µm aufweist.

7. Anschlag nach einem vorhergehenden Anspruch, wobei mindestens eines der Folgenden zutrifft:
die Mikronut (400A) und/oder die Mikrorippe (400P) erstrecken sich in einer Umfangsrichtung;
die Mikronut (400A) und/oder die Mikrorippe (400P) erstrecken sich in einer schraubenförmigen Richtung.

8. Anschlag nach einem der vorhergehenden Ansprüche, wobei die Mikronut (400A) eine Basis und zwei Seiten aufweist und ferner wobei eine der beiden Seiten die Mikrorippe (400P) definiert; optional
wobei das die Mikrorippe (400P) bildende Material eine höhere Dichte aufweist als das die Basis der Mikronut (400A) bildende Material, oder
wobei das die Mikrorippe (400P) bildende Material eine geringere Dichte aufweist als das die Basis der Mikronut (400A) bildende Material.

9. Anschlag nach einem der Ansprüche 1 bis 8, wobei die erste Schicht (402) ein Material beinhaltet, das so konfiguriert ist, dass es sich im Volumen vergrößert, wenn es durch die Energiequelle aktiviert wird,
und wobei ferner die Mikrorippe (400P) einem Abschnitt der ersten Schicht (402) entspricht, dessen Volumen bei Aktivieren durch die Energiequelle vergrößert wurde, oder wobei die erste Schicht (402) ein Material beinhaltet, das so konfiguriert ist, dass es bei Aktivieren durch die Energiequelle entfernt wird, und wobei ferner die Mikronut (400A) einem Abschnitt der ersten Schicht (402) entspricht, der bei Aktivieren durch die Energiequelle entfernt wurde.

10. Anschlag nach einem vorhergehenden Anspruch, wobei die Barriere (242) durch Aktivieren der ersten Schicht (402) mit der Energiequelle an den Elastomerkörper (240) gebunden wurde.

11. Anschlag nach einem vorhergehenden Anspruch, wobei mindestens eines der Folgenden zutrifft:
die Mikronut (400A) und/oder Mikrorippe (400P) wurden nach dem Koppeln der Barriere (242) an den Elastomerkörper (240) ausgebildet;
das Mikromerkmal (400) wird auf der Außenfläche der Barriere (242) ausgebildet;
das Mikromerkmal (400) wird auf der Innenfläche der Barriere (242) ausgebildet;
die Porosität eines Abschnitts der ersten Schicht (402), der der Mikronut (400A) und/oder Mikrorippe (400P) entspricht, wurde nach dem Koppeln der Barriere (242) an den Elastomerkörper (240) modifiziert.

12. Anschlag nach einem der vorhergehenden Ansprüche, wobei das erste Material der ersten Schicht (402) einen Füllstoff beinhaltet, der so konfiguriert ist, dass er die Absorption von Lichtenergie und/oder Hochfrequenzenergie des ersten Materials erhöht, optional
wobei der Füllstoff mindestens eines von fluoriertem Ethylen-Propylen (FEP) und Ethylen-Tetrafluorethylen (ETFE) beinhaltet.

13. Verfahren zur Herstellung eines Anschlags (40) zur Verwendung in einer Injektorvorrichtung (10), wobei der Anschlag (40) eine Dichtungsfläche aufweist, die zum Eingriff mit einer Innenbohrung eines Injektorvorrichtungszylinders (20) konfiguriert ist, wobei das Verfahren umfasst:
Aktivieren einer ersten Schicht (402) einer Barriere (242) des Anschlags (40) mit einer Energiequelle, um eine Volumenänderung der ersten Schicht zu bewirken und mindestens ein Mikromerkmal (400) zu bilden, wobei die Barriere (242) eine Innenfläche und eine Außenfläche aufweist, wobei die Barriere (242) eine erste Schicht (402) aus einem ersten Material und
eine zweite Schicht (404) aus einem zweiten Material beinhaltet, wobei das Mikromerkmal (400) eines oder beide von:
einer Mikronut (400A) und/oder
einer Mikrorippe (400P) beinhaltet; und
Koppeln der Barriere (242) an einen Elastomerkörper (240).

14. Verfahren nach Anspruch 13, wobei mindestens eines der Folgenden zutrifft:
die zweite Schicht (404) ist über der ersten Schicht (402) positioniert, und wobei ferner die erste Schicht (402) durch die zweite Schicht (404) hindurch aktiviert wird;
die erste Schicht (402) beinhaltet FEP und die zweite Schicht (404) beinhaltet PTFE;
die Barriere (242) ist während der Bildung des mindestens einen Mikromerkmals (400) an den Elastomerkörper (240) gekoppelt.

15. Verfahren nach Anspruch 13 oder 14, wobei die Energiequelle mindestens eine von einer Laserenergiequelle, einer HF-Energiequelle, einer Schwingungsenergiequelle und einer thermischen Energiequelle beinhaltet.

16. Verfahren nach einem der Ansprüche 13 bis 15, wobei mindestens eines der Folgenden zutrifft:
Bilden des mindestens einen Mikromerkmals (400) beinhaltet das Kühlen der Barriere (242) nach Aktivieren der ersten Schicht (402);
gleichzeitiges Bilden der Mikronut (400A) und der Mikrorippe (400P), optional durch Bewirken, dass geschmolzene Abschnitte der Barriere (242) wieder fließen und sich wieder verfestigen;
Aktivieren einer ersten Schicht (402) der Barriere (242) des Anschlags (40) mit der Energiequelle, um mindestens ein Mikromerkmal (400) zu bilden, beinhaltet das Induzieren einer Relativbewegung zwischen der Energiequelle und dem Anschlag (40), wobei die Bewegung optional eine oder beide von Linearbewegung oder Drehbewegung beinhaltet.

17. Verfahren nach einem der Ansprüche 13 bis 16, wobei mindestens eines der Folgenden zutrifft:
das mindestens eine Mikromerkmal (400) wird vor dem Koppeln der Barriere (242) an den Elastomerkörper (240) ausgebildet;
das mindestens eine Mikromerkmal (400) wird ausgebildet, wobei die Barriere (242) eine Blattform aufweist;
das mindestens eine Mikromerkmal (400) wird nach dem Koppeln der Barriere (242) an den Elastomerkörper (240) ausgebildet;
das Mikromerkmal (400) wird auf der Außenfläche der Barriere (242) ausgebildet; und das Mikromerkmal (400) wird auf der Innenfläche der Barriere (242) ausgebildet.

## Revendications

1. Bouchon (40) destiné à être utilisé dans un dispositif injecteur (10), le bouchon possédant un côté extérieur conçu pour venir en prise avec un alésage intérieur d'un cylindre de dispositif injecteur (20), le bouchon (40) comprenant :
un corps en élastomère (240) ; et
une barrière (242) couplée au corps en élastomère (240), la barrière (242) possédant une surface interne orientée vers le corps en élastomère (240) et une surface externe orientée à l'opposé du corps en élastomère (240), la barrière (242) comprenant une première couche (402) d'un premier matériau et une seconde couche (404) d'un second matériau, la première couche (402) étant conçue pour être activable par une source d'énergie et la seconde couche (404) étant conçue pour être moins activable par la source d'énergie que la première couche, et :
ladite barrière (242) possédant au moins une micro-fonction (400) formée dans la barrière (242) en activant la première couche (402) avec la source d'énergie afin de provoquer un changement de volume de la première couche lorsqu'elle est activée par la source d'énergie, ladite au moins une micro-fonction (400) comprenant un ou les deux éléments parmi : une micro-rainure (400A) et/ou une micro-nervure (400P).

2. Bouchon (40) selon la revendication 1, ladite source d'énergie comprenant au moins source parmi une source d'énergie laser, une source d'énergie RF, une source d'énergie vibratoire et une source d'énergie thermique.

3. Bouchon (40) selon l'une quelconque des revendications précédentes, au moins l'un de ce qui suit s'appliquant :
ladite première couche (402) est positionnée sous la seconde couche (404) ;
ladite première couche (402) est microporeuse et définit une première porosité et ladite seconde couche (404) possède une porosité inférieure à celle de la première couche ;
ledit premier matériau et/ou ledit second matériau comprend un fluoropolymère, éventuellement du polytétrafluoroéthylène (PTFE) ou du PTFE expansé (PTFEe) ; et
au moins un matériau parmi ledit premier matériau de la première couche (402) et ledit second matériau de la seconde couche (404) comprend un matériau thermoplastique.

4. Bouchon selon l'une quelconque des revendications précédentes, ladite barrière (242) possédant une épaisseur comprise entre 1 µm et 200 µm.

5. Bouchon (40) selon l'une quelconque des revendications précédentes, au moins l'un de ce qui suit s'appliquant :
ladite seconde couche (404) est **caractérisée par** une température de fusion supérieure à celle de la première couche ; et
ladite seconde couche (404) est **caractérisée par** une stabilité dimensionnelle supérieure à celle de la première couche (402).

6. Bouchon (40) selon l'une quelconque des revendications précédentes, ladite micro-rainure (400A) présentant une profondeur de 0,25 µm à 50 µm, et éventuellement de 0,25 µm à 0,5 µm et une largeur de 0,25 µm à 50 µm, et éventuellement de 0,25 µm à 0,5 µm ; et/ou
ladite micro-nervure (400P) présentant une hauteur de 0,25 µm à 50 µm, et éventuellement de 0,25 µm à 0,5 µm et une largeur de 0,25 µm à 50 µm, et éventuellement de 0,25 µm à 0,5 µm.

7. Bouchon selon l'une quelconque des revendications précédentes, au moins l'un de ce qui suit s'appliquant :
ladite micro rainure (400A) et/ou ladite micro nervure (400P) s'étend suivant une direction circonférentielle ;
ladite micro-rainure (400A) et/ou ladite micro-nervure (400P) s'étend suivant une direction hélicoïdale.

8. Bouchon selon l'une quelconque des revendications précédentes, ladite micro-rainure (400A) possédant une base et deux côtés, et en outre l'un desdits deux côtés définissant la micro- nervure (400P) ; éventuellement
ledit matériau formant la micro-nervure (400P) présentant une densité plus élevée que le matériau formant la base de la micro-rainure (400A), ou
ledit matériau formant la micro nervure (400P) présentant une densité inférieure à celle du matériau formant la base de la micro-rainure (400A).

9. Bouchon selon l'une quelconque des revendications 1 à 8, ladite première couche (402) comprenant un matériau conçu pour augmenter de volume lorsqu'il est activé par la source d'énergie,
et en outre ladite micro-nervure (400P) correspondant à une partie de la première couche (402) qui a subi une augmentation de volume en étant activée par la source d'énergie, ou ladite première couche (402) comprenant un matériau conçu pour être retiré lorsqu'il est activé par la source d'énergie, et en outre ladite micro-rainure (400A) correspondant à une partie de la première couche (402) qui a été retirée en étant activée par la source d'énergie.

10. Bouchon selon l'une quelconque des revendications précédentes, ladite barrière (242) ayant été liée au corps en élastomère (240) en activant la première couche (402) avec la source d'énergie.

11. Bouchon selon l'une quelconque des revendications précédentes, au moins l'un de ce qui suit s'appliquant :
ladite micro rainure (400A) et/ou ladite micro-nervure (400P) a été formée suite au couplage de la barrière (242) au corps en élastomère (240) ;
ladite micro-fonction (400) est formée sur la surface externe de la barrière (242) ;
ladite micro-fonction (400) est formée sur la surface interne de la barrière (242) ;
ladite porosité d'une partie de la première couche (402) correspond à la micro-rainure (400A) et/ou à la micro-nervure (400P) a été modifiée suite au couplage de la barrière (242) au corps en élastomère (240).

12. Bouchon selon l'une quelconque des revendications précédentes, ledit premier matériau de la première couche (402) comprenant une charge conçue pour augmenter l'absorption de l'énergie lumineuse et/ou de l'énergie radiofréquence du premier matériau, éventuellement
ladite charge comprenant au moins un élément parmi l'éthylène-propylène fluoré (FEP) et l'éthylène-tétrafluoroéthylène (ETFE).

13. Procédé de fabrication d'un bouchon (40) destiné à être utilisé dans un dispositif injecteur (10), le bouchon (40) possédant une surface d'étanchéité conçue pour venir en prise avec un alésage intérieur d'un cylindre de dispositif injecteur (20), le procédé comprenant :
l'activation d'une première couche (402) d'une barrière (242) du bouchon (40) avec une source d'énergie de manière à provoquer un changement de volume de la première couche et à former au moins une micro-fonction (400), la barrière (242) possédant une surface interne et une surface externe, la barrière (242) comprenant une première couche (402) d'un premier matériau et
une seconde couche (404) d'un second matériau, la micro-fonction (400) comprenant un ou les deux éléments parmi : une micro-rainure (400A) et/ou
une micro nervure (400P) ; et
le couplage de la barrière (242) à un corps en élastomère (240).

14. Procédé selon la revendication 13, au moins l'un de ce qui suit s'appliquant :
ladite seconde couche (404) est positionnée sur la première couche (402), et en outre ladite première couche (402) est activée à travers la seconde couche (404) ;
ladite première couche (402) comprend du FEP et ladite seconde couche (404) comprend du PTFE ;
ladite barrière (242) est couplée au corps en élastomère (240) pendant la formation de ladite micro-fonction (400).

15. Procédé selon les revendications 13 ou 14, ladite source d'énergie comprenant au moins une source parmi une source d'énergie laser, une source d'énergie RF, une source d'énergie vibratoire et une source d'énergie thermique.

16. Procédé selon l'une quelconque des revendications 13 à 15, au moins l'un de ce qui suit s'appliquant :
ladite formation de ladite au moins une micro-fonction (400) comprend le refroidissement de la barrière (242) après activation de la première couche (402) ;
ladite formation simultanée de la micro-rainure (400A) et de la micro-nervure (400P), éventuellement en provoquant la refusion et la resolidification des parties fondues de la barrière (242) ;
ladite activation d'une première couche (402) de la barrière (242) du bouchon (40) avec la source d'énergie afin de former au moins une micro-fonction (400) comprend l'induction d'un déplacement relatif entre la source d'énergie et le bouchon (40), ledit déplacement comprenant éventuellement un ou les deux déplacements parmi un déplacement linéaire ou un déplacement de rotation.

17. Procédé selon l'une quelconque des revendications 13 à 16, au moins l'un de ce qui suit s'appliquant :
ladite au moins une micro-fonction (400) est formée avant le couplage de la barrière (242) au corps en élastomère (240) ;
ladite au moins une micro-fonction (400) est formée avec la barrière (242) sous forme de feuille ;
ladite au moins une micro-fonction (400) est formée après le couplage de la barrière (242) au corps en élastomère (240) ;
ladite micro-fonction (400) est formée sur la surface externe de la barrière (242) ; et ladite micro-fonction (400) est formée sur la surface interne de la barrière (242).
